(19) Europäisches Patentamt
European Patent Office
Office européen des brevets

(11) Veröffentlichungsnummer : **0 456 608 A2**

# (12) EUROPÄISCHE PATENTANMELDUNG

(21) Anmeldenummer : **91810330.0**

(22) Anmeldetag : **30.04.91**

(51) Int. Cl.[5] : **C08G 65/40**, C08G 61/12, C08G 63/193, C08G 64/00, C08G 69/40, C08G 75/23

(30) Priorität : **08.05.90 CH 1555/90**

(43) Veröffentlichungstag der Anmeldung : **13.11.91 Patentblatt 91/46**

(84) Benannte Vertragsstaaten : **DE ES FR GB IT NL SE**

(71) Anmelder : **CIBA-GEIGY AG**
**Klybeckstrasse 141**
**CH-4002 Basel (CH)**

(72) Erfinder : **Pfaendner, Rudolf, Dr.**
**Sackgasse 3**
**W-6149 Rimbach/Odenwald 1 (DE)**
Erfinder : **Kainmüller, Thomas, Dr.**
**Am Kümmelberg 1**
**W-6145 Lindenfels 1 (DE)**
Erfinder : **Hoffmann, Kurt, Dr.**
**Heidenbergstrasse 15**
**W-6147 Lautertal 2 (DE)**
Erfinder : **Stockinger, Friedrich**
**Au Fernotz**
**CH-1784 Courtepin (CH)**
Erfinder : **Kramer, Andreas, Dr.**
**Bundtels**
**CH-3186 Düdingen (CH)**

(54) **Im wesentlichen lineare Polymere.**

(57) Im wesentlichen lineare Polymere mit einer reduzierten Viskosität von 0,1 bis 2,0 dl/g, gemessen an einer 1 gew.-%igen Lösung in N-Methylpyrrolidon (NMP) bei 25°C, enthaltend mindestens einen die Löslichkeit des Polymeren in organischen Lösungsmitteln erhöhenden Anteil an Strukturεinheiten der Formel I

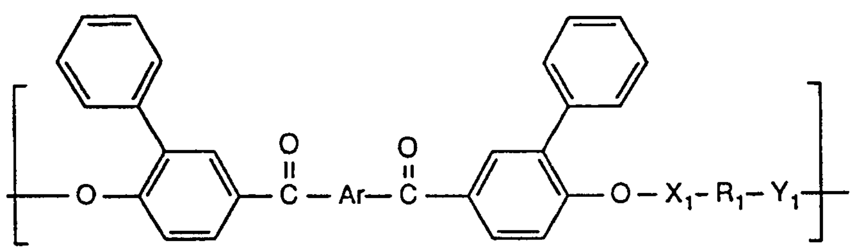

(I),

worin die aromatischen Ringe unsubstituiert oder substituiert sind, Ar einen zweiwertigen aliphatischen, cycloaliphatischen, araliphatischen oder aromatischen Rest mit bis zu 50 C-Aomen bedeutet,
$X_1$ für eine direkte Bindung, $-SO_2-$, -CO-, -CO-O-, -CO-NH- oder

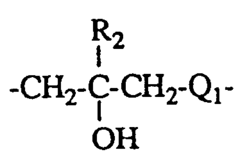

steht, worin $R_2$ ein H-Atom oder Methyl und $Q_1$ -O-, -NH-, -S- oder -OCO- bedeuten,
$Y_1$ für eine direkte Bindung, $-SO_2-$, -CO-, -O-CO-, -NH-CO- oder

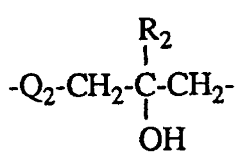

steht, worin $R_2$ ein H-Atom oder Methyl und $Q_2$ -O-, -NH-, -S- oder -OCO- bedeuten, $R_1$ einen

EP 0 456 608 A2

zweiwertigen aliphatischen, cycloaliphatischen, aromatischen oder araliphatischen Rest mit bis zu 30 C-Atomen bedeutet, können vorteilhaft aus der Lösung heraus verarbeitet werden und eignen sich zum Modifizieren von Matrixharzen.

Die vorliegende Erfindung betrifft im wesentlichen lineare Polymere mit Arylenetherketoneinheiten aus neuen 4,4'-Dihalogen- oder 4,4'-Dihydroxy-3,3'-diphenylphthalophenonen, Verfahren zu deren Herstellung, die aus den erfindungsgemässen Polymeren hergestellten Formstoffe, Beschichtungen oder Folien sowie die Verwendung der erfindungsgemässen Polymeren zum Modifizieren von Matrixharzen.

Polyarylenetherketone und Polyarylenethersulfone sind technische Werkstoffe mit sehr guten mechanischen und thermischen Eigenschaften, die eine hohe Beständigkeit gegenüber herkömmlichen organischen Lösungsmitteln aufweisen. Für manche Anwendungen ist jedoch diese Lösungsmittelresistenz nicht erwünscht, insbesondere sollen bei der Modifizierung von duromeren Matrixharzen möglichst konzentrierte Polymerlösungen in üblichen organischen Lösungsmitteln zur Verwendung kommen.

Polyarylenetherketone aus unsubstituierten Dihydroxyiso- oder -terephthalophenon sind aus der DE-OS 30 14 230 bekannt. Aufgrund des Einbaues von Iso- oder Terephthalophenon-Einheiten weisen die dort beschriebenen Polyarylenetherketone eine verbesserte Lösungsmittelbeständigkeit gegenüber den gebräuchlichen organischen Lösungsmitteln, beispielsweise Methylenchlorid, Aceton oder Chlorbenzol, auf.

Es wurde nun gefunden, dass Polymere mit Arylenetherketoneinheiten aus 4,4'-Dihydroxy-3,3'-diphenylphthalophenonen überraschend in gebräuchlichen organischen Lösungsmitteln, vorzugsweise in halogenierten Kohlenwasserstoffen oder cyclischen Ketonen, gut löslich sind und stabile Lösungen bilden.

Gegenstand der vorliegenden Erfindung sind somit im wesentlichen lineare Polymere mit einer reduzierten Viskosität von 0,1 bis 2,0 dl/g, gemessen an einer 1 gew.-%igen Lösung in N-Methylpyrrolidon (NMP) bei 25°C, enthaltend mindestens einen die Löslichkeit des Polymeren in organischen Lösungsmitteln erhöhenden Anteil an Struktureinheiten der Formel I

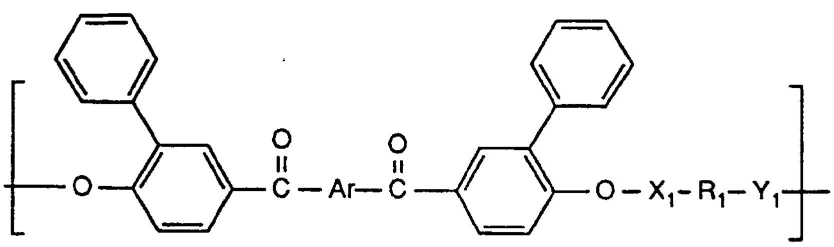

(I),

worin die aromatischen Ringe unsubstituiert oder durch ein oder mehrere $C_1$-$C_4$-Alkyle, $C_1$-$C_4$-Alkoxy oder Halogenatome substituiert sind,

Ar einen zweiwertigen aliphatischen, cycloaliphatischen, araliphatischen oder aromatischen Rest mit bis zu 50 C-Atomen bedeutet,

$X_1$ für eine direkte Bindung, $-SO_2-$, -CO-, -CO-O-, -CO-NH- oder

$$-CH_2-\underset{OH}{\overset{R_2}{C}}-CH_2-Q_1-$$

steht, worin $R_2$ ein H-Atom oder Methyl und $Q_1$ -O-, -NH-, -S- oder -OCO- bedeuten,

$Y_1$ für eine direkte Bindung, $-SO_2-$, -CO-, -O-CO-, -NH-CO- oder

$$-Q_2-CH_2-\underset{OH}{\overset{R_2}{C}}-CH_2-$$

steht, worin $R_2$ ein H-Atom oder Methyl und $Q_2$ -O-, -NH-, -S- oder -OCO- bedeuten,

$R_1$ einen zweiwertigen aliphatischen, cycloaliphatischen, aromatischen oder araliphatischen Rest mit bis zu 30 C-Atomen bedeutet, der unsubstituiert ist oder durch ein oder mehrere $C_1$-$C_4$-Alkyle, $C_1$-$C_4$-Alkylthio, $C_2$-$C_6$-Alkenyle, $C_5$-$C_{12}$-Cycloalkyle, $C_6$-$C_{12}$-Aryle oder eine oder mehrere Cyano- oder Nitrogruppen substituiert ist und in dem ein oder mehrere Kohlenstoffatome durch Sauerstoff-, Schwefel- oder Stickstoffatome ersetzt sein können.

Der Begriff "im wesentlichen linear" ist so zu verstehen, dass die erfindungsgemässen Polymeren entweder

lineare Ketten ausbilden oder einen geringen Verzweigungsgrad aufweisen, so dass sie sich ohne Schwierigkeit in einem herkömmlichen organischen Lösungsmittel, beispielsweise in einem chlorierten Kohlenwasserstoff oder in einem polaren aprotischen Lösungsmittel, lösen lassen.

Die Löslichkeit wird im allgemeinen vom Anteil der Struktureinheiten der Formel I beeinflusst werden. Dabei wird die Löslichkeit mit zunehmender Konzentration dieser Struktureinheiten in der Regel zunehmen. Unter "einem die Löslichkeit des Polymeren erhöhenden Anteil an Struktureinheiten der Formel I" ist im Falle von Copolymeren im Rahmen dieser Beschreibung zumindest ein solcher Anteil zu verstehen, der im Vergleich zu nicht mit solchen Struktureinheiten modifizerten Polymeren (Basispolymer) entweder überhaupt erst zu einer Löslichkeit in einem bestimmten Lösungsmittel führt oder der zu einer merklichen Erhöhung der Lagerstabilität einer Lösung führt. Uebliche Werte für die Konzentration einer Polymerlösung bewegen sich im Bereich von 1-75 Gew.%, insbesondere von 5-50 Gew.% des Polymeren, bezogen auf die Lösung.

Die für ein bestimmtes Polymer zum Erzielen einer gewünschten Löslichkeit in einem vorgegebenen Lösungsmittel benötigte Menge an Struktureinheiten der Formel I kann mittels Routineversuchen ermittelt werden. In der Regel enthalten die erfindungsgemässen Polymeren mehr als 5 Mol-%, vorzugsweise mehr als 10 Mol-%, und insbesondere mehr als 20 Mol-% dieser Struktureinheiten, bezogen auf das Polymere.

Unter dem Begriff "Polymer" sind im Rahmen dieser Beschreibung auch Oligomere zu verstehen. Die reduzierte Viskosität der erfindungsgemässen Polymeren erstreckt sich über einen Bereich von etwa 0,1 bis etwa 2,0 dl/g. Dies entspricht einem Molekulargewichtsbereich von etwa 1'000 bis etwa 100'000 (Zahlenmittel). Bevorzugt werden Polymere mit einem Zahlenmittel des Molekulargewichts von 5'000 bis 50'000. Im Falle der Diglycidylether auf Basis der erfindungsgemässen Bisphenole können die Oligomeren auch Molekulargewichte wesentlich unterhalb von 1'000 aufweisen.

Der Rest Ar in der Struktureinheit der Formel I steht vorzugsweise für einen aromatischen Rest, besonders bevorzugt für einen unsubstituierten oder durch ein oder mehrere $C_1$-$C_4$-Alkyle, $C_1$-$C_4$-Alkoxy oder Halogenatome substituierten Rest der Formeln

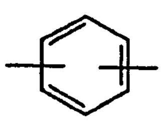 , 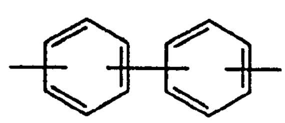 , 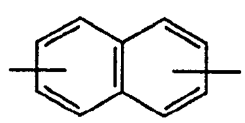 , 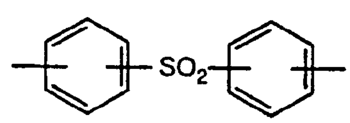 ,

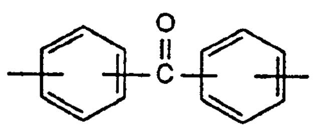 oder 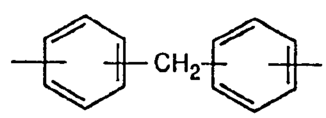 ,

insbesondere für einen unsubstituierten definitionsgemässen Rest, ganz besonders für einen 1,3- oder 1,4-Phenylenrest.

Die Reste $X_1$ und $Y_1$ in den Struktureinheiten der Formel I können im Rahmen der gegebenen Definitionen gleiche oder unterschiedliche Bedeutungen aufweisen. Vorzugsweise haben diese Reste die gleiche Bedeutung, und insbesondere stehen $X_1$ und $Y_1$ für eine direkte Bindung.

Der Rest $R_1$ bedeutet vorzugsweise einen unsubstituierten oder substituierten cycloaliparischen oder aromatischen Rest, insbesondere einen aromatischen Rest.

Enthalten die aromatischen Ringe in der Struktureinheit der Formel I, der Rest Ar oder der Rest $R_1$ in Formel I Alkylsubstituenten, so können diese Methyl, Ethyl, n-Propyl, Isopropyl, n-Butyl, sek.-Butyl oder tert.-Butyl sein.

Beispiele für Alkoxyreste sind Methoxy, Ethoxy, n-Propoxy, Isopropoxy, n-Butoxy und tert.-Butoxy.

Bedeuten irgendwelche Reste Halogenatome, so handelt es sich hierbei im allgemeinen um Chlor, Brom, Fluor oder Jod, vorzugsweise um Chlor oder Brom.

Beispiele für Alkylthioreste sind Methylthio, Ethylthio, n-Propylthio, Isopropylthio oder n-Butylthio.

Bedeuten irgendwelche Reste Alkenyl, so handelt es sich dabei um verzweigte oder insbesondere um geradkettige Reste. Alkenylreste weisen zwei bis sechs Kohlenstoffatome auf. Beispiele für Alkenylreste sind Vinyl, Prop-1-enyl, Prop-2-enyl, n-But-3-enyl, n-Pent-4-enyl oder n-Hex-5-enyl. Bevorzugt werden geradkettige Alkenylreste mit zwei oder drei Kohlenstoffatomen, insbesondere Vinyl, Prop-1-enyl oder Prop-2-enyl (Allyl). Alkenylsubstituenten werden besonders bevorzugt, da sie eine Vernetzung der erfindungsgemässen Polymeren über die Alkenylgruppen gestatten.

Bedeuten irgendwelche Reste Cycloalkyl, so handelt es sich dabei im allgemeinen um Gruppen mit fünf bis acht Ringkohlenstoffatomen. Bevorzugt wird Cyclohexyl. Beispiele für Cycloalkylreste sind Cyclopentyl, Cyclohexyl, Cycloheptyl oder Cyclooctyl.

Beispiele für Arylreste sind Phenyl, Naphthyl und Biphenyl, vorzugsweise handelt es sich hierbei um Phenyl.

Im Rest $R_1$ können auch ein oder mehrere, vorzugsweise ein, zwei oder drei Kohlenstoffatome durch Sauerstoff-, Schwefel- und/oder Stickstoffatome ersetzt sein. Beispiele für derartig modifizierte Alkylenketten sind Reste, die sich von Polyalkylenglykolen ableiten. Ferner kann es sich um aromatische oder nicht aromatische heterocyclische Systeme handeln, die vorzugsweise fünf- oder sechsgliedrig sind. Im Falle von heterocyclischen Systemen sind vorzugsweise ein bis drei Ringkohlenstoffatome durch Stickstoffatome oder ein oder zwei Ringkohlenstoffatome durch Sauerstoff- oder Schwefelatome ersetzt. Es können auch unterschiedliche Heteroatome in einem Ring auftreten, beispielsweise ein Stickstoff- und ein Sauerstoffatom.

Beispiele für heterocyclische Reste sind Morpholindiyl, Piperidindiyl, Furandiyl, Pyridindiyl, Thiophendiyl, Triazindiyl und Pyridazindiyl.

Die Definition "ein oder mehrere Kohlenstoffatome sind durch Sauerstoff-, Schwefel- und/oder Stickstoffatome ersetzt" umfasst auch solche heterocyclischen Systeme, bei denen die Ringheteroatome neben der Einbindung in das Ringsystem noch ihrerseits zusätzliche Atome oder Substituenten tragen. Beispiele für solche Heterogruppen sind $-SO_2-$, -NH- oder -NO-.

Die erfindungsgemässen Polymeren weisen entweder gleiche oder unterschiedliche wiederkehrende Struktureinheiten auf oder es handelt sich um Copolymere, die neben der wiederkehrenden Struktureinheit der Formel I noch wiederkehrende Struktureinheiten der Formel II

$$\{O-R_3-O-X_1-R_1-Y_1\} \quad (II)$$

aufweisen, worin $X_1$, $R_1$ und $Y_1$ die gleiche Bedeutung wie in Formel I haben und $R_3$ einen zweiwertigen aliphatischen, cycloaliphatischen, araliphatischen oder aromatischen Rest mit bis zu 50 C-Atomen bedeutet, der vom Rest

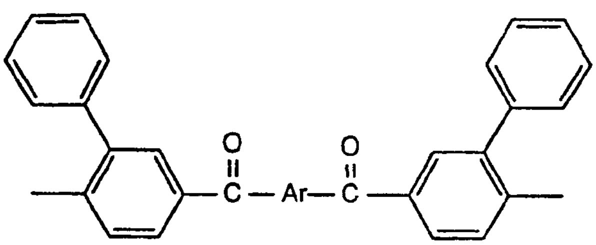

verschieden ist, worin Ar die gleiche Bedeutung wie in Formel I hat.

Die bevorzugten erfindungsgemässen Polymeren enthalten, bezogen auf die Gesamtmenge der im Polymer vorhandenen Struktureinheiten, 5 bis 100 Mol-%, insbesondere 10 bis 100 Mol-%, einer wiederkehrenden Struktureinheit der Formel I und 95 bis 0 Mol-%, insbesondere 90 bis 0 Mol-%, einer wiederkehrenden Struktureinheit der Formel II.

Vorzugsweise bedeutet $R_3$ einen unsubstituierten oder durch ein oder mehrere $C_1$-$C_4$-Alkyle, $C_1$-$C_4$-Alkoxy oder Halogenatome substituierten Rest der Formeln IIIa bis IIIg

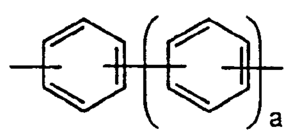 (IIIa),

wobei a Null oder die Zahl 1 ist,

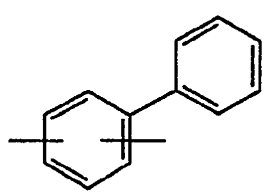 (IIIb), 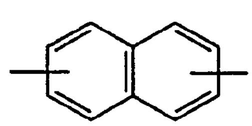 (IIIc),

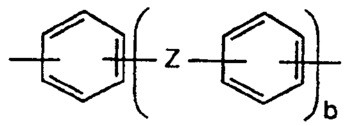 (IIId),

wobei b die Zahl 1 oder 2 ist,

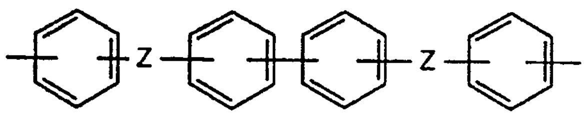 (IIIe), 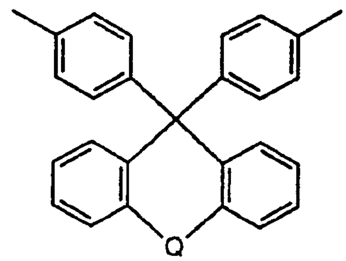 (IIIf)

oder 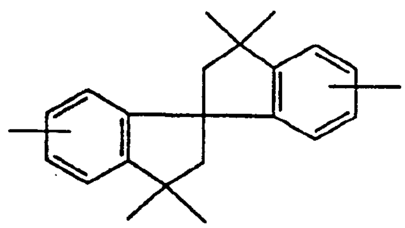 (IIIg)

darstellt, worin Z für -CO-, $-SO_2-$, -SO-, -S-, -O-,

$$-\overset{|}{C}(CH_3)_2\ ,\quad -\overset{|}{C}(CF_3)_2\ ,$$

$-CH_2-$,

$$-\underset{\underset{C_6H_5}{|}}{\overset{|}{C}}-CH_3 \text{ oder } -\underset{\underset{R}{|}}{\overset{|}{P}}O$$

steht, wobei R ein Methyl oder Phenyl bedeutet, und Q für $-CH_2-$, -O-,

$$-\overset{\overset{O}{\|}}{C}-$$

oder die direkte Bindung steht.

Insbesondere bedeutet $R_3$ einen Rest der Formeln

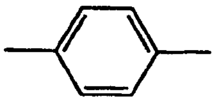 , 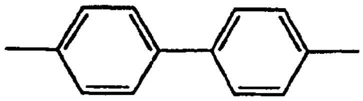 , 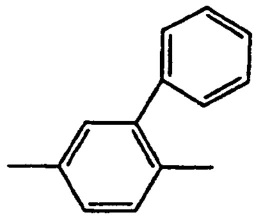 ,

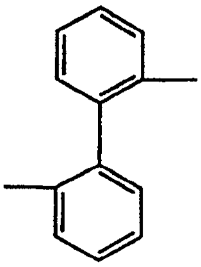 , 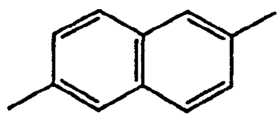 , 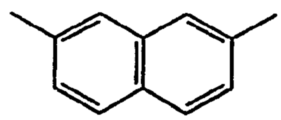 ,

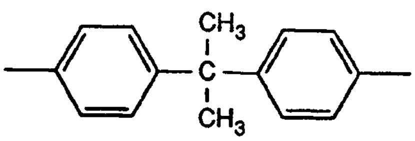 , 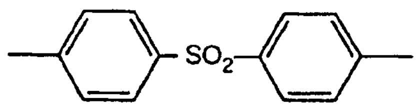 ,

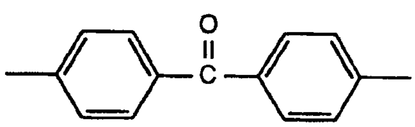 , 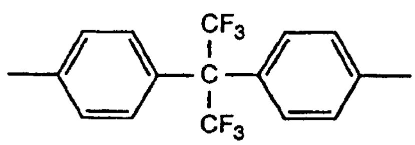 ,

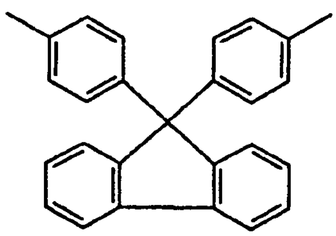 oder 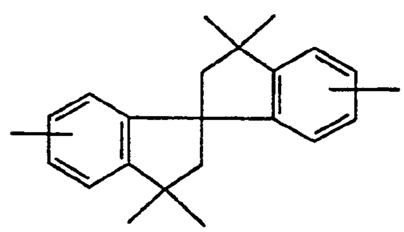 . Und ganz besonders steht $R_3$ für

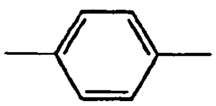 , 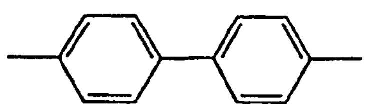 , 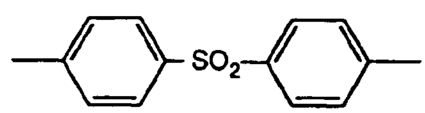 ,

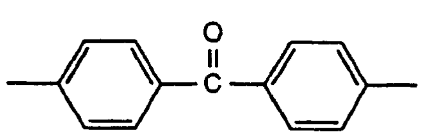 oder 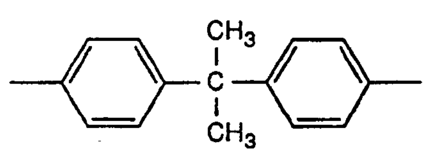 .

Beispiele für erfindungsgemässe Polymere sind Polyarylenether, wie Polyarylenetherketone und Polyarylenethersulfone, Polysulfonate, Polycarbonate, Polyester, Polyurethane und Phenoxyharze, wobei diese noch mit anderen Monomeren, die sich mit den betreffenden Monomeren der Polymeren copolymerisieren lassen, modifiziert werden können. Ausserdem können die Endgruppen der erfindungsgemässen Polymeren, wie beispielsweise die Hydroxyl- oder Carboxylgruppen in bekannter Weise weiter umgesetzt werden, beispielsweise mit Epichlorhydrin oder ß-Methylepichlorhydrin, wobei nach der Dehydrochlorierung die entsprechenden Diglycidylverbindungen erhalten werden. Desgleichen lassen sich die erfindungsgemässen Polymeren mit Diaminen oder Dianhydriden modifizieren.

Besonders bevorzugte Polymere dieser Erfindung sind überwiegend aus aromatischen Gruppen aufgebaut. Davon sind besonders die Polyether und davon die Polyetherketone und Polyethersulfone bevorzugt. Erfindungsgemässe Polymere mit aromatischen Gruppen zeichnen sich in der Regel durch besonders gute thermische Eigenschaften aus. Dabei handelt es sich bevorzugt um Verbindungen, die zu 10 bis 100 Mol% aus wiederkehrenden Struktureinheiten der Formel I bestehen.

Ganz besonders bevorzugte Polymere dieser Erfindung sind Polyarylenether, insbesondere solche, die, bezogen auf die Gesamtmenge der im Polyarylenetherharz vorhandenen Struktureinheiten, 5-100 Mol-% einer wiederkehrenden Struktureinheit der Formel IV

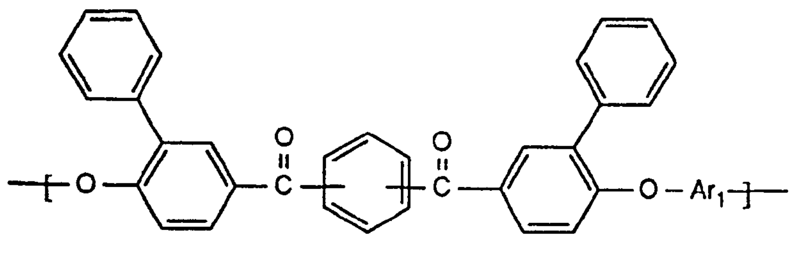 (IV)

und 95-0 Mol-% einer wiederkehrenden Struktureinheit der Formel V

$\{O-Ar_2-O-Ar_1\}$ (V)

enthalten, worin die beiden Carbonylgruppen zueinander in para- oder meta-Stellung stehen, $Ar_1$ einen unsubstituierten oder durch ein oder mehrere $C_1$-$C_4$-Alkyle, $C_1$-$C_4$-Alkoxy oder Halogenatome substituierten Rest der Formel IVa bis IVe

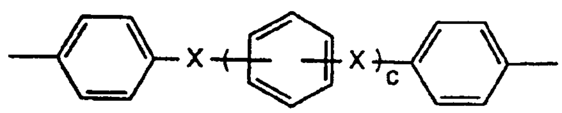 (IVa),

wobei c für Null oder die Zahl 1 oder 2 steht, oder

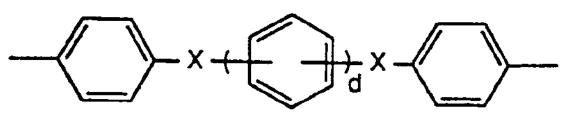 (IVb),

wobei d die Zahl 2 oder 3 bedeutet,

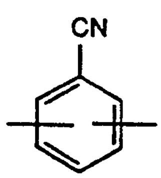 (IVc), 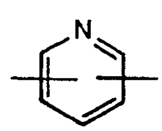 (IVd) oder 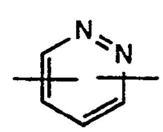 (IVe)

darstellt, worin X für -CO-, $-SO_2-$ oder -SO- steht, und $Ar_2$ einen unsubstituierten oder durch ein oder mehrere $C_1$-$C_4$-Alkyle, $C_1$-$C_4$-Alkoxy oder Halogene substituierten Rest der Formeln Va-Vg

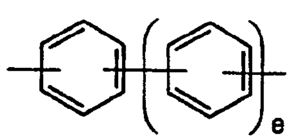 (Va),

wobei e Null oder die Zahl 1 bedeutet,

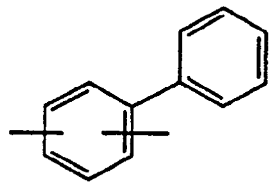 (Vb), 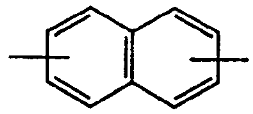 (Vc),

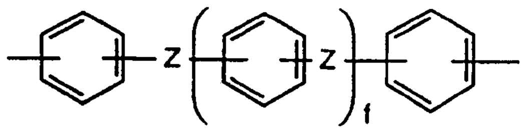 (Vd),

wobei f Null oder die Zahl 1 ist,

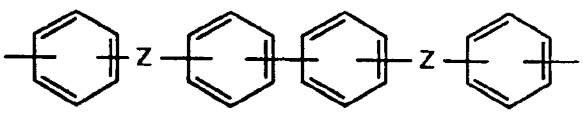 (Ve), 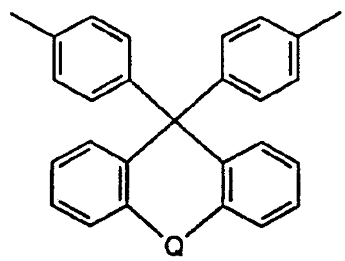 (Vf)

oder

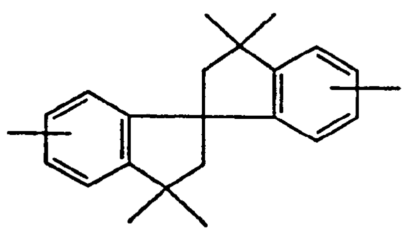 (Vg)

darstellt, worin Z für -CO-, $-SO_2-$, -SO-, -S-, -O-,

$$-\overset{|}{C}(CH_3)_2\ ,\quad -\overset{|}{C}(CF_3)_2\ ,$$

$-CH_2-$,

$$-\underset{C_6H_5}{\overset{|}{\underset{|}{C}}}-CH_3 \text{ oder } -\underset{R}{\overset{|}{\underset{|}{P}}}O$$

steht, worin R ein Methyl oder Phenyl bedeutet und Q für $-CH_2-$, -O-,

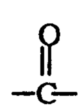

oder die direkte Bindung steht.

In den erfindungsgemässen Polyarylenethern sind die Strukturelemente der Formeln IV und V vorzugsweise unsubstituiert.

Vorzugsweise enthalten die erfindungsgemässen Polyarylenether 100-10 Mol-% einer wiederkehrenden Struktureinheit der Formel IV und 90-0 Mol-% einer wiederkehrenden Struktweinheit der Formel V, worin die beiden Carbonylgruppen zueinander in meta- oder para-Stellung stehen, $Ar_1$ vorzugsweise einen Rest der Formel

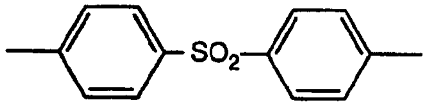 , 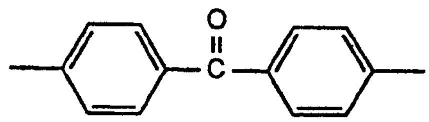

oder

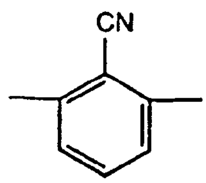

bedeutet und $Ar_2$ vorzugsweise für einen Rest der Formeln

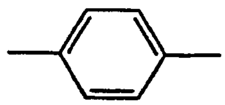 , 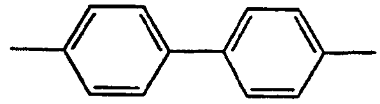 , 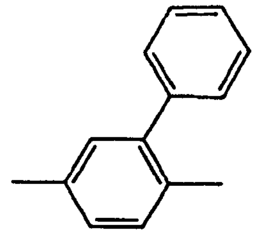 ,

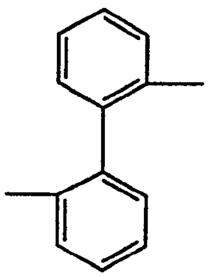 , 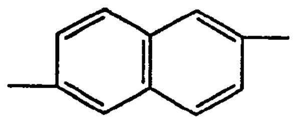 , 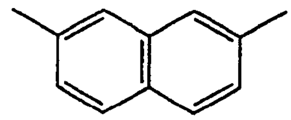 ,

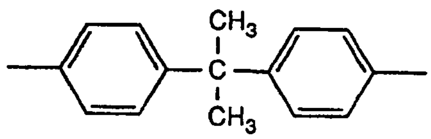 , 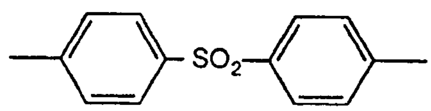 ,

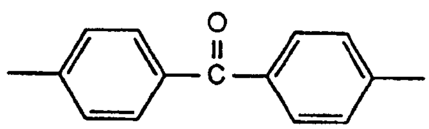 , 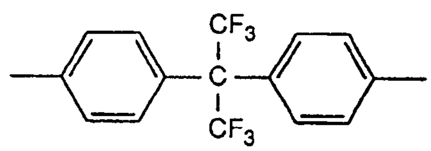 ,

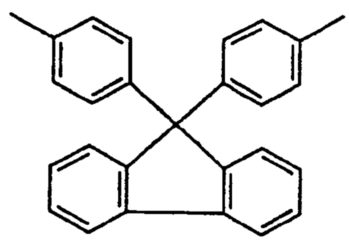 oder 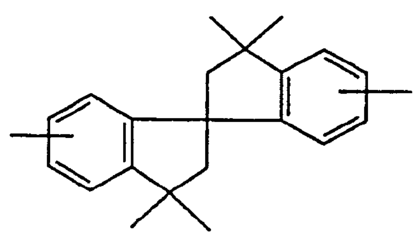 steht.

Die erfindungsgemässen im wesentlichen linearen Polymeren, enthaltend Struktureinheiten der Formel I, können hergestellt werden, indem man ein unsubstituiertes oder durch ein oder mehrere $C_1$-$C_4$-Alkyle, $C_1$-$C_4$-Alkoxy oder Halogenatome substituiertes Diphenol der Formel VI

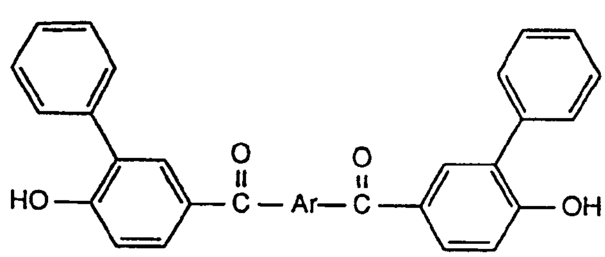 (VI),

worin Ar die gleiche Bedeutung wie in Formel I hat, oder ein Gemisch aus einem Diphenol der Formel VI und einer darin bis zu 95 Mol-% enthaltenen, vom Diphenol der Formel VI verschiedenen Dihydroxyverbindung in äquimolaren Mengen mit einer Verbindung der Formel VII

$$Z_1\text{-}R_1\text{-}Z_2 \qquad \text{(VII)},$$

umsetzt, worin $R_1$ die gleiche Bedeutung wie in Formel I hat und $Z_1$ und $Z_2$ unabhängig voneinander je ein Halogenatom, vorzugsweise Fluor- oder Chloratom, oder einen Rest der Formeln $-SO_3H$, $-SO_3R_4$, $-SO_2Cl$, -CO-OH, -COCl, $-COOR_4$, $-OR_4$, -O-COCl, -N=C=O,

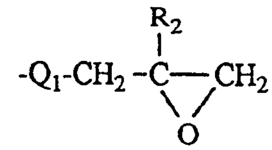 oder 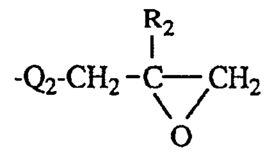

bedeuten, worin $R_2$ für ein H-Atom oder Methyl und $R_4$ für ein $C_1$-$C_4$-Alkyl oder Phenyl stehen und $Q_1$ und $Q_2$ -O-, -NH-, -S- oder -OCO- bedeuten.

Unter äquimolaren Mengen versteht man im Zusammenhang mit dem oben angegebenen Verfahren ein Molverhältnis der Diphenolverbindung der Formel VI oder des Gemisches von Diphenol- und Dihydroxyverbindung zu der Verbindung der Formel VII von 0,9 bis 1, 1. Bevorzugt ist ein Molverhältnis von 0,95 bis 1,05.

Die Verbindungen der Formel VI sind noch nicht in der Literatur beschrieben worden und stellen somit einen weiteren Erfindungsgegenstand dar.

Vorzugsweise steht Ar in Formel VI für einen unsubstituierten Rest der Formeln

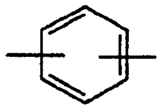 , 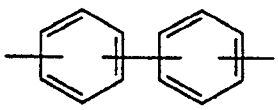 , 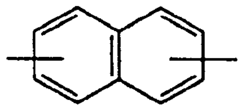 , 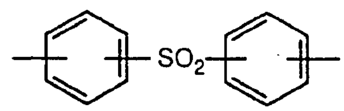 ,

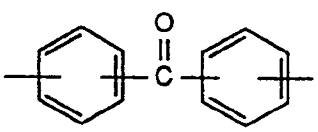 oder 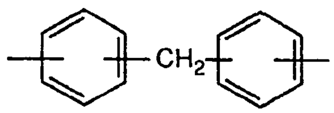 ,

insbesondere für 1,3- oder 1,4-Phenylen

Die Verbindungen der Formel VI können hergestellt werden, indem man 1 Mol einer aromatischen Dicarbonsäure oder eines aromatischen Dicarbonsäuredichlorids der Formel VIII

$$X_1\text{-CO-Ar-CO-}X_1 \qquad (VIII),$$

worin $X_1$ je für ein -OH oder ein Chloratom steht und Ar die gleiche Bedeutung wie in Formel I hat, mit 2 Mol eines unsubstituierten oder durch ein oder mehrere $C_1$-$C_4$-Alkyle, $C_1$-$C_4$-Alkoxy oder Halogenatome substituierten 2-Alkoxybiphenyls(2-Methoxybiphenyl) in Gegenwart von $FeCl_3$ als Katalysator im Temperaturbereich von 80-160°C umsetzt und die erhaltene unsubstituierte oder substituierte Verbindung der Formel IX

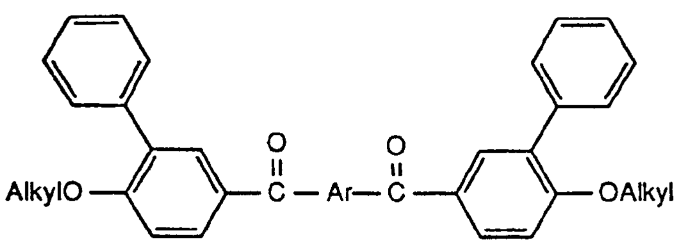 (IX),

worin Ar die gleiche Bedeutung wie in Formel I hat und Alkyl für ein $C_1$-$C_4$-Alkyl steht, mit der 2- bis 6-fachen Gewichtsmenge $AlCl_3$, bezogen auf die Gewichtsmenge der Verbindung der Formel IX, im Temperaturbereich von 40-100°C umsetzt, wobei eine Verbindung der Formel VI erhalten wird.

Die Verbindungen der Formel IX sind ebenfalls noch nicht in der Literatur beschrieben worden und stellen somit einen weiteren Erfindungsgegenstand dar.

Als Dihydroxyverbindungen, die von denen der Formel VI verschieden sind, eignen sich im allgemeinen aliphatische, cycloaliphatische und aromatische Dihydroxyverbindungen. Beispiele für geeignete Dihydroxyverbindungen sind:

Ethylenglykol, Diethylenglykol, Propan-1,2-diol, Propan- 1,3-diol, Butan-1,4-diol, Poly-(oxyethylenglykol), Poly-(oxypropylenglykol), Poly-(oxybutylenglykol), Pentan-1,5-diol, Hexan-1,6-diol, Octan-1,8-diol, 2,2-Dimethylpropan-1,3-diol, trans-Tetramethylcyclobutandiol und 1,4-Bis-(hydroxymethyl)-cyclohexan, 1,1-Bis- (hydroxymethyl)-cyclo-hex-3-en, Bis-(4-hydroxycyclohexyl)-methan, 2,2-Bis-(4-hydroxycyclohexyl)-propan, Hydrochinon, Chlor-, Methyl-, 2,3-Dimethyl- oder Trimethylhydrochinon, Tetrafluorhydrochinon, Resorcin, 1,2-Bis-(4-hydroxyphenyl)-ethan, 2,2-Bis-(4-hydroxyphenyl)-propan (Bisphenol A), Tetrabrombisphenol A, Hexafluorbisphenol A, Bis-(4-hydroxyphenyl)-ether, Bis-(4-hydroxyphenyl)-sulfid, Bis-(4-hydroxyphenyl)-sulfon, Bis-(hydroxyphenyl)-methan (Bisphenol F), Bis-(4-hydroxy-3,5-dimethylphenyl)-methan, 2,2-Bis-(4-hydroxy-3-methylphenyl)-propan, 2,2-Bis-(4-hydroxy-3,5-dimethylphenyl)-propan, 2,2-Bis-(4-hydroxy-3,5-dichlorphenyl)-propan, Bis-(4-hydroxy-3,5-dimethylphenyl)-sulfid, Bis-(4-hydroxy-3,5-dimethylphenyl)-sulfon, 2,2-Bis- (4-hydroxy-3,5-dibromphenyl)-propan, 4,4′-Dihydroxybiphenyl, 4,4′-Dihydroxy-2,2′-dimethylbiphenyl, 2,2′-Dihydroxybiphenyl, 2,5 -Dihydroxybiphenyl, 4,4′-Dihydroxyterphenyl, 4,4′-Dihydroxybenzophenon, 2,2′-Dihydroxybenzophenon, 4,4′-Dihydroxydiazo-biphenyl, 4,4′-Dihydroxyterephthalophenon, 4,4′-Dihydroxyisophthalophenon, 4,4′-Dihydroxytriphenylmethan, Phenolphthalein, Phenolsulfonphthalein, Fluorescein, 9,9-Bis-(4-hydroxyphenyl)-fluoren, 1,5-Dihydroxyanthrachinon, 2,6-Dihydroxyanthrachinon, 1,5-Dihydroxynaphthalin, 1,6-Dihydroxynaphthalin, 2,6-Dihydroxynaphthalin, 2,7-Dihydroxynaphthalin, 4,5-Bis-(4-hydroxyphenyl)-2-phenylimidazol und 2,3-Bis-(4-hydroxyphenyl)-chinoxalin. Solche Dihydroxyverbindungen sind bekannt und zum Teil im Handel erhältlich.

Beispiele für geeignete polyetherbildende Halogenverbindungen der Formel VIII sind: 1,3-Dibrombenzol, 1,4-Dibrombenzol, 4,4′-Dibrombiphenyl, 4,4′-Dibrombenzophenon, 2,7-Dibrom-9-fluorenon, 1,3-Bis-(chlormethyl)-benzol, 1,4-Bis-(chlormethyl)-benzol, 1,4-Bis-(brommethyl)-benzol, 1,5-Bis-(chlormethyl)-naphthalin, Bis-(4-chlorphenyl)-sulfon, Bis-(4-fluorphenyl)-sulfon, Bis-(4-chlorphenyl)-keton, Bis-(4-fluorphenyl)-keton, 2,6-Difluorbenzonitril, 2,6-Dichlorpydin, 4,6-Di-chlor-2-phenyl-1,3,5-triazin, 4,6-Di-chlor-2-methyl- 1,3,5-triazin, 4,6-Di-chlor-2-methoxy-1,3,5-triazin und 3,6-Dichlorpyridazin. Diese stellen ebenfalls bekannte Verbindung dar und sind kommerziell erhältlich.

Beispiele für geeignete Disulfonsäure(derivate) der Formel VII sind:
1,3-Benzoldisulfonsäure, 1,4-Benzoldisulfonsäure, 4,4′-Biphenyidisulfonsäure, 4,4′-Oxy-bis-(benzolsulfonsäure), 4,4′-Sulfonyl-bis-(benzolsulfonsäure), 4,4′-Methylen-bis-(benzolsulfonsäure) und 2,2-Bis-(benzolsulfonsäure)-propan und die Disulfonsäuredichloride, die Disulfonsäuredialkylester mit 1 bis 4 C-Atomen im Alkylrest, der Disulfonsäurediphenylester und die Dinatriumsalze dieser Säuren. Auch diese Verbindungen sind bekannt.

Beispiele für geeignete polycarbonatbildende Verbindungen der Formel VII sind Phosgen, Chlorameisensäurealkyl- oder -phenylester, Kohlensäuredialkyl- oder -diphenylester oder Verbindungen der Formeln Cl-CO-O-$R_1$-O-COCl oder $R_4$O-CO-O-$R_1$-O-CO-O$R_4$, worin $R_1$ und $R_4$ die gleiche Bedeutung wie in Formel I haben. Solche Verbindungen sind bekannt und zum Teil im Handel erhältlich.

Beispiele für geeignete Dicarbonsäuren oder deren Polyester bildende Derivate der Formel VII sind:
Adipinsäure, Azelainsäure, Sebacinsäure, Suberinsäure, Pimelinsäure, Hexahydroterephthalsäure, Terephthalsäure, Isophthalsäure, 2,5-Dichlorterephthalsäure, 2,5-Dichlorisophthalsäure, 2-Phenylisophthalsäure, 5-Methylisophthalsäure, 4,4′-Biphenyldicarbonsäure, Diphenylsulfon-4,4′-dicarbonsäure, Diphenylether- 4,4′-dicarbonsäure, Diphenylether-3,4′-dicarbonsäure, Diazodiphenyl-4,4′-dicarbonsäure, Diphenylmethan-4,4′-dicarbonsäure, 2,2-Bis-(4-benzoesäure)-propan, 2,2-Bis-(4-benzoesäure)-hexafluoropropan, Triphenylphosphinoxid-4,4é-dicarbonsäure, Diphenylmethylphosphinoxid-4,4é-dicarbonsäure, Naphthalin-2,6- dicarbonsäure, 1,4-Cyclohexandicarbonsäure, [2.2.2]-Bicyclooctandicarbonsäure und [3.2.2]- Bicyclononandicarbonsäure, sowie die Methyl-, Ethyl- oder Phenylester dieser Säuren oder deren Säurechloride. Auch diese bekannten Verbindungen sind zum Teil im Handel erhältlich.

Beispiele für geeignete Diisocyanate der Formel VII sind:
Hexamethylendiisocyanat, Cyclohexan-1,4-diisocyanat, 2,4- und 2,6-Toluylendiisocyanat und deren Gemische, Isophorondiisocyanat (1-Isocyanatomethyl-5-isocyanato-1,3,3-trimethyl-cyclohexan), Phorondiisocyanat (2,2,4- bzw. 2,4,4-Trimethyl-hexamethylendiisocyanat-1,6), 1,5-Naphthalindiisocyanat, 1,3-Cyclopentylendiisocyanat, m- und p-Phenylendiisocyanat, 2,4,6-Toluylentriisocyanat, 4,4′,4″-Triphenylmethantriisocyanat, 1,3- und 1,4-Xylylendiisocyanat, 3,3′-Dimethyl-4,4′-diphenylmethandiisocyanat, 4,4′-Diphenyl-methandiisocyanat, 3,3′-Dimethylbiphenylendiisocyanat, 4,4′-Bisphenylendiisocyanat, Durendiisocyanat, 1-Phenoxy-2,4′-phenylendiisocyanat, 1-tert.-Butyl-2,4-phenylendiisocyanat, Methylen-bis-4,4′-cyclohexyldiisocyanat, 1-Chlor-2,4-phenylendiisocyanat und 4,4′-Diphenyletherdiisocyanat, welche bekannte Verbindungen darstellen.

Zur Herstellung der Phenoxyharze eignen sich als Diglycidylverbindungen der Formel VII beispielsweise die Diglycidylether und Diglycidylester der oben aufgezählten Dihydroxyverbindungen und Dicarbonsäuren. Ferner können auch Di-S-glycidylverbindungen, wie beispielsweise Ethan-1,2-dithioldiglycidylether oder Bis-(4-glycidylthiomethylphenyl)-ether, oder Di-N-glycidylverbindungen, beispielsweise unsubstituiertes oder alkylsubstituiertes N,N′-Diglycidyl-5,5-dimethylhydantoin, als Diglycidylverbindungen der Formel VII eingesetzt werden. Vorzugsweise verwendet man als Diglycidylverbindung einen Diglycidylether eines Bisphenols, insbesondere des Bisphenols A oder F. Diese Diglycidylverbindungen sind ebenfalls bekannt und zum Teil im Handel erhältlich.

Ausserdem können als Verbindung der Formel VII auch solche mit verschiedenen funktionellen Gruppen verwendet werden, beispielsweise Isocyanatocarbonsäurehalogenide, wie 4-Isocyanatophenylcarbonsäurechlorid, Isocyanatosulfonsäurehalogenide, wie 4-Isocyanatophenylsulfonsäurechlorid, oder Halogencarbonyloxycarbonsäurehalogenide, wie 4-Chlorcarbonyloxyphenylcarbonsäurechlorid.

Wie bereits erwähnt wurde, können die erfindungsgemässen Polymeren noch mit anderen Monomeren, die sich mit den Monomeren der Polymeren copolymerisieren lassen, modifiziert sein. Solche Monomere sind beispielsweise Hydroxycarbonsäuren, Hydroxysulfonsäuren oder Aminophenole.

Beispiele für geeignete Hydroxycarbonsäuren oder deren polyesterbildende Derivate sind:
4-Hydroxybenzoesäure, 3-Chlor-4-hydroxybenzoesäure, 3,5-Dichlor-4-hydroxybenzoesäure, 3-Methyl-4-hydroxybenzoesäure, 3-Brom-4-hydroxybenzoesäure, 3,5-Dimethyl-4-hydroxybenzoesäure, 3-Methoxy-4-hydroxybenzoesäure, 6-Hydroxy-2-naphthoesäure, 6-Hydroxy-5-chlor-2-naphthoesäure und 6-Hydroxy- 5-methyl-2-naphthoesäure sowie die Methyl-, Ethyl- oder Phenylester dieser Säuren oder deren Säurechloride, sowie die Monoester dieser Phenole, wie die Acetate.

Beispiele für geeignete Hydroxysulfonsäuren oder deren polyesterbildende Derivate sind die Analogen der oben aufgezählten Hydroxycarbonsäure(derivate), bei denen die Carboxylgruppe durch eine Sulfonsäuregruppe ersetzt ist.

Beispiele für geeignete Aminophenole sind:
3-oder 4-Aminophenol, 2-Hydroxy-5-amino-naphthalin, 2-Hydroxy-6-amino-naphthalin oder 2-Hydroxy-7-amino-naphthalin.

Die Herstellung der besonders bevorzugten Polyarylenether erfolgt beispielsweise durch
(a) Polykondensation eines Diphenols der Formel VI, eines Gemisches aus einem Diphenol der Formel VI oder dessen Alkali- oder Erdalkaliphenolat und einer darin bis zu 95 Mol-% enthaltenen Dihydroxyverbindung der Formel X

$$HO\text{-}Ar_2\text{-}OH \qquad (X)$$

oder deren Alkali- oder Erdalkalisalze, worin $Ar_2$ die gleiche Bedeutung wie in Formel V hat, mit einer äquimolaren Menge einer Dihalogenverbindung der Formel VII, worin $Z_1$ und $Z_2$ je für Fluor oder Chlor stehen, in Gegenwart von Alkali in einem aprotischen Lösungsmittel oder durch
(b) Polykondensation einer unsubstituierten oder durch ein oder mehrere $C_1$-$C_4$-Alkyle, $C_1$-$C_4$-Alkoxy oder Halogenatome substituierten Dihalogenverbindung der Formel XI

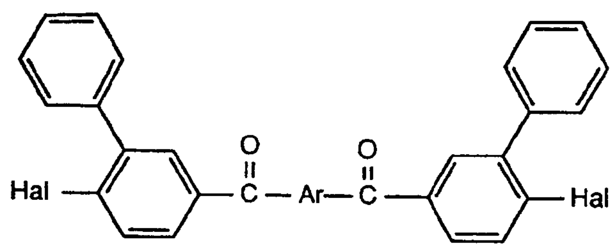
(XI),

worin Ar die gleiche Bedeutung wie in Formel I hat und Hal für ein Halogenatom, vorzugsweise Fluor- oder Chloratom, steht, oder eines Gemisches aus einer Dihalogenverbindung der Formel XI und einer darin bis zu 95 Mol-% enthaltenen, von der Dihalogenverbindung der Formel XI verschiedenen Dihalogenverbindung der Formel VII in äquimolaren Mengen mit einer Dihydroxyverbindung der Formel X oder deren Alkalioder Erdalkalisalze in Gegenwart von Alkali in einem aprotischen Lösungsmittel, bis der erhaltene Polyarylenether eine reduzierte Viskosität von 0,1 bis 2,0 dl/g, vorzugsweise von 0,15 bis 1,8 dl/g insbesondere von 0,2 bis 1,5 dl/g, aufweist, gemessen an einer 1 gew.%igen Lösung in N-Methylpyrrolidon (1 g Polyarylenether in 100 ml N-Methylpyrrolidon) bei 25°C.

Als Alkali verwendet man in diesem Verfahren in der Regel Alkali- und Erdalkalicarbonate, wie Natrium-, Kalium- oder Calciumcarbonat; doch können auch andere alkalische Reagentien, wie Natriumhydroxid, Kaliumhydroxid oder Calciumhydroxid, Verwendung finden.

Polare, aprotische Lösungsmittel, die beim Verfahren zur Herstellung der erfindungsgemässen Polyetherharze eingesetzt werden können, sind beispielsweise Dimethylsulfoxid, Dimethylacetamid, Diethylacetamid, Tetramethylharnstoff, N-Methylcaprolactam, N-Methylpyirolidon und bevorzugt Diphenylsulfon.

Die Reaktion wird zweckmässig bei erhöhter Temperatur durchgeführt, vorzugsweise bis zur Rückflusstemperatur des Lösungsmittels, also etwa bis 350°C.

Häufig empfiehlt sich die Mitverwendung eines Schleppmittels, wie z.B. Chlorbenzol, Xylol oder Toluol, um das bei der Umsetzung gebildete Wasser azeotrop aus dem Reaktionsgemisch entfemen zu können.

Die Verbindungen der Formel XI sind in der Literatur noch nicht beschrieben worden und stellen einen weiteren Erfindungsgegenstand dar.

Die Verbindungen der Formel XI können hergestellt werden, indem man 1 Mol einer aromatischen Dicarbonsäwe oder eines aromatischen Dicarbonsäuredichlorids der Formel VIII

$X_1$-CO-Ar-CO-$X_1$ (VIII),

worin $X_1$ je für -OH oder ein Chloratom stehen und Ar die gleiche Bedeutung wie in Formel I hat, mit 2 Mol eines unsubstituierten oder durch ein oder mehrere $C_1$-$C_4$-Alkyle, $C_1$-$C_4$-Alkoxy oder Halogenatome substituierten 2-Fluorbiphenyls in Gegenwart von $FeCl_3$ als Katalysator im Temperaturbereich von 80-160°C zu einer Verbindung der Formel XI umsetzt.

Die Herstellung der erfindungsgemässen Polymeren, die durch Umsetzung eines Diphenols der Formel VI mit einer Disulfonsäure, einem Disulfonsäurediester oder -dichlorid erhalten werden, kann in der Schmelze oder in Lösung, beispielsweise als Grenzflächenpolykondensation in einem inerten Lösungsmittel und in Gegenwart einer Base, beispielsweise von Kaliumcarbonat oder -hydroxid, erfolgen.

Die Herstellung der erfindungsgemässen Polymeren, die durch Umsetzung eines Diphenols der Formel VI mit einer Dicarbonsäure oder deren polyesterbildenden Derivaten und gegebenenfalls weiteren Dihydroxyverbindungen und/oder Aminoalkoholen und/oder Hydroxycarbonsäuren und/oder Hydroxysulfonsäuren oder den polyesterbildenden Derivaten dieser Säuren erhalten werden, kann beispielsweise durch Umesterung von geeigneten Dicarbonsäureestern mit Diphenolen, sowie durch Veresterung der freien Säure mit Diphenolderivaten, wie z.B. den Diacetaten, in der Schmelze und gegebenenfalls in Gegenwart von basischen oder sauren Katalysatoren erfolgen. Geeignete Katalysatoren sind Dibutylzinnoxid, p-Toluolsulfonsäure, Bortrifluorid, Titandioxid, Calciumacetat, Manganacetat, Zinkacetat oder Antimonoxid. Die Herstellung der Polyester kann auch durch Grenzflächenpolykondensation oder durch Polykondensation in einem inerten Lösungsmittel erfolgen. Je nach der Aktivität der eingesetzten Monomeren kann die Polykondensation bei erhöhten Temperaturen oder bei Temperaturen bis unterhalb Raumtemperatur durchgeführt werden.

Die Herstellung der erfindungsgemässen Polymeren, die durch Polyaddition eines Diphenols der Formel VI mit einer Diglycidylverbindung erhalten werden, kann ebenfalls in der Schmelze als auch in Lösung erfolgen. Die Herstellung von sogenannten Phenoxyharzen ist an sich bekannt und wird beispielsweise im DE-Patent 1 935 115 beschrieben.

Die Herstellung der erfindungsgemässen Polycarbonate erfolgt beispielsweise durch Umesterung von Diphenylcarbonat mit Bisphenolen der Formel VI in der Schmelze und in Gegenwart von basischen Katalysatoren, wie LiOH oder NaOH, oder durch Umsetzung von Bisphenolen der Formel VI mit Phosgen oder einer Bis-chlorcarbonylverbindung in einem inerten Lösungsmittel. Die Umsetzung mit Phosgen oder einer Bis-chlorcarbonylverbindung kann auch durch Grenzflächenpolykondensation des Phenolats in einem Gemisch aus Wasser und einem inerten Lösungsmittel erfolgen.

Die Herstellung von erfindungsgemässen Polymeren, die durch stufenweise Polyaddition von Diisocyanaten an Verbindungen der Formel VI erhalten werden, wird nach den in der Polyurethanchemie üblichen Verfahren durchgeführt. Als Katalysatoren verwendet man meist tert. Amine oder zinnorganische Verbindungen.

Weitere erfindungsgemässe Verbindungen, welche sich von dem Diphenol der Formel VI ableiten, stellen die Diglycidylether der Formel XII

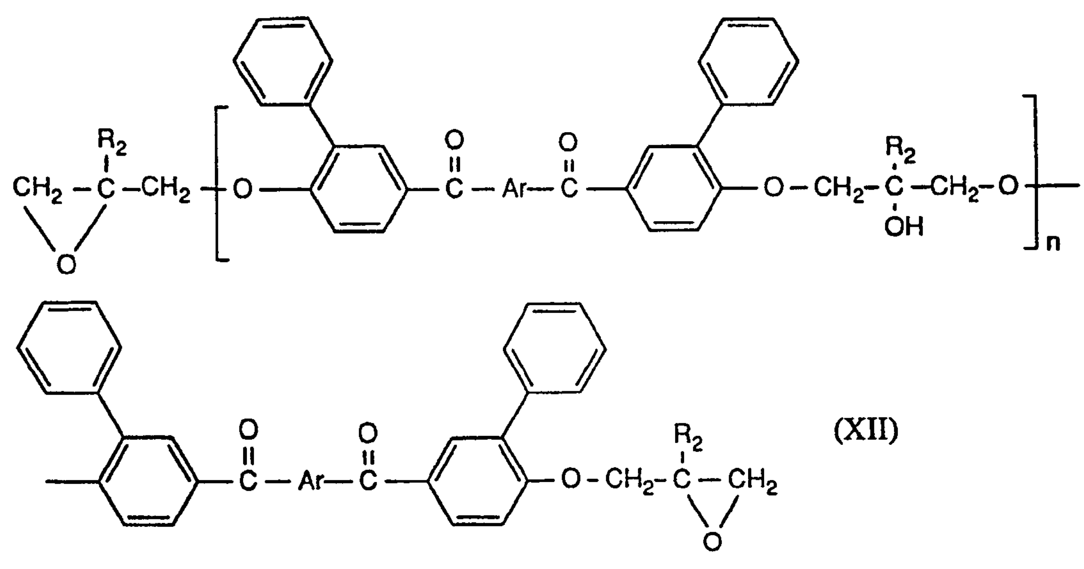

(XII)

dar, worin Ar die gleiche Bedeutung wie in Formel I hat, $R_2$ für ein H-Atom oder Methyl steht und n Null oder eine Zahl von 1 bis 20 ist.

Solche Diglycidylether werden in bekannter Weise durch Umsetzen eines Diphenols der Formel VI mit Epichlorhydrin oder ß-Methylepichlorhydrin in Gegenwart von Natronlauge und anschliessende Dehydrochlorierung des in Zwischenstufe erhaltenen Chlorhydrinethers erhalten. Verfahren zur Herstellung von Diglycidylethern werden beispielsweise im "Handbook of Epoxy Resins" von H. Lee und K. Neville (Mc Graw-Hill Book Company, 1967), im Kapitel 2, Seite 3 ff näher beschrieben.

Die erfindungsgemässen Polymeren, insbesondere die aromatischen Polyether, Polyetherketone oder Polyethersulfone, können in der für Thermoplaste üblichen Weise eingesetzt werden und z.B. zu Formkörpern oder Folien verarbeitet werden, oder als Matrixharze, Klebstoffe oder Ueberzugsmittel eingesetzt werden.

Die erfindungsgemässen Diglycidylether können in der für Epoxidharze üblichen Weise eingesetzt werden. Sie eignen sich vor allem in Kombination mit an sich üblichen Härtern und gegebenenfalls Härtungsbeschleunigern zur Herstellung von vernetzten Produkten. Gegebenenfalls setzt man auch Gemische der erfindungsgemässen Diglycidylether mit anderen an sich üblichen Epoxidharzen ein.

Beispiele für Epoxidhärter sind Polyamine mit mindestens zwei primären und/oder sekundären Aminogruppen, Amide einschliesslich der substituierten Harnstoffe, Polyaminoamide, Polyphenole, Polythiole, Polycarbonsäuren und insbesondere deren Anhydride, katalytisch wirkende Härtungsmittel, wie beispielsweise tertiäre Amine, Imidazole oder Mannichbasen, Zinnsalze von Alkansäuren, Friedel-Crafts-Katalysatoren und deren Komplexe und Chelate, oder Amidine, wie beispielsweise Dicyandiamid.

Beispiele für Härtungsbeschleuniger sind tertiäre Amine, deren Salze oder quaternäre Ammoniumverbindungen oder substituierte Harnstoffe. Diese Härter und Härtungsbeschleuniger sind dem Fachmann auf dem Gebiet der Epoxidverarbeitung an sich bekannt und beispielsweise im "Handbook of Epoxy Resins " von Lee and Neville beschrieben.

Vor der Verarbeitung der beispielsweise als Presspulver, Schmelze oder insbesondere als Lösung vorliegenden Polymeren können übliche Zusatzstoffe, wie beispielsweise Füllstoffe, Pigmente, Stabilisatoren oder Verstärkungsmittel, wie Kohlenstoff-, Bor-, Metall- oder Glasfasern, zugegeben werden. Die erfindungsgemässen Polymeren können auch zusammen mit anderen Thermoplasten oder Duromeren verarbeitet werden.

Vorzugsweise eignen sich die erfindungsgemässen Polymeren, insbesondere die aromatischen Polyether, Polyetherketone oder Polyethersulfone, als Matrixharze für die Herstellung von Faserverbundsystemen, wobei man als Verstärkungsfasern die üblichen bei der Verstärkung technischer Werkstoffe verwendeten Fasern einsetzen kann. Diese können organische oder anorganische Fasern, Naturfasern oder Synthesefasern sein, und als Faserbündel, als orientierte oder nicht-orientierte Fasern oder als Endlosfasern vorliegen.

Eine weitere bevorzugte Anwendungsmöglichkeit für die erfindungsgemässen Polymeren, insbesondere für die aromatischen Polyether, Polyetherketone oder Polyethersulfone, besteht in der Modifizierung anderer Kunststoffe. Diese können grundsätzlich Thermoplaste oder Duromere sein. Besondere Bedeutung erlangen solche Systeme als Matrixharze, welche zur Herstellung von Verbundbauteilen zum Einsatz gelangen.

Besonders hervorzuheben ist die unerwartet hohe Löslichkeit der erfindungsgemässen Polymeren in einer Reihe von herkömmlichen organischen Lösungsmitteln, wie in fluorierten oder chlorierten Kohlenwasserstoffen, und die sehr gute Stabilität dieser Lösungen. Beispiele für geeignete Lösungsmittel sind polare aprotische Lösungsmittel, wie N-Methylpyrrolidon, N,N-Dimethylformamid, Dimethylsulfoxid und Sulfolan, fluorierte oder chlorierte aliphatische und aromatische Kohlenwasserstoffe, wie Methylenchlorid, Tri-oder Tetra-chlorethan, 1,2-Dichlorethan oder Chlorbenzol, oder cyclische Ether, wie Tetrahydrofuran, 1,3-Dioxolan oder Dioxan, sowie auch Cyclohexanon oder Cyclopentanon.

Die Erfindung betrifft daher auch eine Lösung enthaltend etwa 1 bis 75 Gew.%, vorzugsweise 5 bis 50 Gew.%, bezogen auf die Lösung, eines erfindungsgemässen Polymers, vorzugsweise eines erfindungsgemässen aromatischen Polymers, insbesondere eines erfindungsgemässen Polyarylenetherketons oder Polyarylenethersulfons gelöst in einem organischen Lösungsmittel.

<u>Herstellung von Ausgangsstoffen</u>

<u>Beispiel A:</u> 4,4'-Dimethoxy-3,3'-diphenylisophthalophenon

In einem 1,5 Liter Sulfierkolben, ausgerüstet mit Thermometer, Rührer, Kühler und Stickstoffspülung, werden 555,0 g (3,0124 Mol) 2-Methoxybiphenyl, 61,1 g (0,30124 Mol) Isophthalsäuredichlorid und 1,14 g (0,00708 Mol) Eisen-(III)-chlorid 2 Stunden und 20 Minuten lang bei 150°C zur Reaktion gebracht. Anschliessend werden 35,4 g 2n NaOH innerhalb von 25 Minuten zugetropft, 30 Minuten bei 109°C gerührt und danach auf 40°C abgekühlt. Das Reaktionsgemisch wird filtriert, der Filterrückstand mit je 150 ml Wasser und Methylenchlorid gewaschen, das zweiphasige Filtrat getrennt und die organische Phase 6 mal mit Wasser gewaschen. Man trocknet die Methylenchloridphase mit Natriumsulfat, filtriert und engt das Filtrat anschliessend am Rotationsverdampfer ein. Das überschüssige 2-Methoxybiphenyl wird abdestilliert und man erhält 97,8 g (65,1 % der Theorie) eines orange-roten Pulvers, das heiss in Isopropanol umkristallisiert wird. Es werden 81,32 g (54,2 % der Theorie)

4,4'-Dimethoxy-3,3'-diphenylisophthalphenon als Kristalle isoliert, deren Schmelzpunkt 110°C beträgt.

| Elementaranalyse: | berechnet | gefunden |
|---|---|---|
| | C = 81,91 % | C = 81,48 % |
| | H = 5,26 % | H = 5,39 %. |

Beispiel B: 4,4'-Dimethoxy-3,3'-diphenylterephthalophenon

Analog Beispiel A werden 450,0 g (2,442 Mol) 2-Methoxybiphenyl, 49,5 g (0,2442 Mol) Terephthalsäuredichlorid und 0,92 g (0,0057 Mol) Eisen-(III)-chlorid zur Reaktion gebracht. Nach der Zugabe von 28,7 g 2n NaOH wird das Reaktionsgemisch 30 Minuten lang bei 110°C erwärmt, danach auf Raumtemperatur abgekühlt, die Suspension abfiltriert und der Rückstand mit je 200 ml Wasser und Methylenchlorid gewaschen, trockengesaugt und der feuchte Filterrückstand in 1 Liter Dimethylformamid umkristallisiert.
Man erhält 87,0 g (71,5 % der Theorie) 4,4'-Dimethoxy-3,3'-diphenylterephthalophenon mit einem Schmelzpunkt von 267°C.

| Elementaranalyse: | berechnet | gefunden |
|---|---|---|
| | C = 81,91 % | C = 82,02 % |
| | H = 5,26 % | H = 5,37 %. |

Beispiel C: 4,4'-Dihydroxy-3,3'-diphenylisophthalophenon

In einer Rührapparatur, bestehend aus einem 2,5-Liter Sulfierkolben, Rührer, Thermometer, Kühler und Stickstoffspülung, werden 150 g (0,3008 Mol) 4,4'-Dimethoxy-3,3'-diphenylisophthalophenon, hergestellt gemäss Beispiel A, in 1 Liter Toluol vorgelegt und innerhalb von 15 Minuten bei 24°C -32°C Innentemperatur portionenweise unter Rühren mit 221,4 g (1,6604 Mol) Aluminiumchlorid versetzt und anschliessend 2 Stunden und 5 Minuten lang bei 75°C reagieren gelassen. Das Reaktionsgemisch wird auf Raumtemperatur abgekühlt, mit 10 Liter HCl-saurem Wasser vermischt, die wässrige Phase 4 mal mit je 400 ml Essigsäureethylester extrahiert, die vereinigten organischen Phase 5 mal mit je 500 ml Wasser gewaschen und getrennt, die organische Phase mit Natriumsulfat getrocknet, filtriert und das Filtrat am Rotationsverdampfer bei 90°C und Wasserstrahlvakuum eingeengt und bei 130°C/0,2 mbar getrocknet.
Man erhält 141,4 g (99,9 % der Theorie) 4,4'-Dihydroxy-3,3'-diphenylisophthalophenon als rotes, kristallines Pulver, das in Essigester umkristallisiert wird. Das gereinigte Produkt wird in 72 %-iger Reinausbeute isoliert und hat einen Schmelzpunkt von 169°C.

| Elementaranalyse: | berechnet | gefunden |
|---|---|---|
| | C = 81,69 % | C = 81,71 % |
| | H = 4,71 % | H = 4,76 %. |

Das durch potentiometrische Titration ermittelte Aequivalentgewicht beträgt 237.

Beispiel D: 4,4'-Dihydroxy-3,3'-diphenylterephthalophenon

85 g (0,175 Mol)4,4'-Dimethoxy-3,3'-diphenylterephthalophenon, hergestellt gemäss Beispiel B, und 125,5 g (0,9411 Mol) Aliminiumchlorid werden in 580 ml Toluol analog Beispiel C umgesetzt und aufgearbeitet.
Man erhält 80 g Rohprodukt, das in 300 g 5%-iger wässriger NaOH warm gelöst und anschliessend über Hyflo® filtriert wird. Das Filtrat wird unter intensivem Rühren in eine Lösung von 2 Liter Wasser und 37 g 37 %-iger Salzsäure eingetragen, das ausgefällte Produkt wird abfiltriert, mit Wasser gewaschen und der Filterrückstand wird bei 130°C im Vakuum getrocknet.
Es werden 70,5 g (88,1 % der Theorie) 4,4'-Dihydroxy-3,3'-diphenylterephthalophenon als farbloses, kristallines Produkt erhalten, das bei 303°C schmilzt.

Das durch potentiometrische Titration ermittelte Aequivalentgewicht beträgt 238 (Theorie: 235,26). Die

Reinheit HPLC) beträgt 99,6 %.

Beispiel 1: Polyetherketonsulfon aus 4,4'-Dihydroxy-3,3'-diphenylisophthalophenon und 4,4'-Dichlordiphenylsulfon

In einem Rundkolben mit Rührer und Schutzgasanschluss wird unter Stickstoff eine Mischung aus 18,49 g (0,0502 Mol) 4,4'-Dihydroxy-3,3'-diphenylisophthalophenon, 49,60 g Diphenylsulfon, 7,38 g (0,0534 Mol) Kaliumcarbonat und 56 g Xylol bei einer Badtemperatur von 200°C erhitzt und ein Xylol/Wasser-Gemisch abdestilliert. Gegen Ende des Destillationsvorganges wird dabei kurzzeitig Vakuum (2 mbar) angelegt. Sodann werden 14,38 g (0,0501 Mol) 4,4'-Dichlordiphenylsulfon zu der Reaktionsmischung gegeben, die Temperatur innerhalb von 25 Minuten auf 250°C erhöht und dort 1 Stunde belassen. Danach wird die Temperatur auf 275°C (1 h) und anschliessend auf 300°C angehoben. Diese Temperatur wird 4 Stunden beibehalten, wobei die Rektionsmischung in zunehmendem Masse viskos wird.

Nach Abkühlen wird das Rektionsgemisch dem Kolben entnommen, pulverisiert, mit verdünnter Essigsäure versetzt und zunächst mit Wasser und dann mit einem Wasser/Aceton-Gemisch (1:4) extrahiert. Anschliessend wird das Polymer in Methylenchlorid gelöst, eine geringe Menge unlösliches Material abfiltriert und in Isopropanol ausgefällt. Das so gereinigte Polymer wird im Vakuumtrockenschrank bis zu einer Temperatur von 240°C getrocknet. Ein auf diese Weise hergestelltes Polyarylenetherketon besitzt eine reduzierte Viskosität (1 g Polymeres in 100 ml NMP bei 25°C) von 0, 12 dl/g. Es ist zu mehr als 25 Gew.-% in Methylenchlorid löslich. Die durch Differential Scanning Calorimetry (DSC) bestimmte Glasübergangstemperatur liegt bei 173°C.

Beispiel 2: Polyetherketonsulfon-Copolymer aus 4,4'-Dihydroxy-3,3'-diphenylisophthalophenon, 4,4'-Dihydroxydiphenylsulfon und 4,4-Dichlordiphenylsulfon

In analoger Weise wie in Beispiel 1 wird ein Polyethersulfon aus 4,4'-Dihydroxy-3,3'-diphenylisophthalophenon (0,0251 Mol), 4,4'-Dihydroxydiphenylsulfon (0,0752 Mol) und 4,4'-Dichlordiphenylsulfon (0,100 Mol) mit Kaliumcarbonat (0,1050 Mol) hergestellt (Reaktionsbedingungen: 1 h/250°C, 1 h/275°C, 3 h/300°C). Das resultierende Polymer mit einer red. Viskosität von 0,42 dl/g besitzt eine Glasübergangstemperatur von 215°C. Es ist zu mehr als 25 % in Methylenchlorid löslich.

Beispiel 3: Polyetherketonsulfon-Copolymer aus 4,4'-Dihydroxy-3,3'-diphenylisophthalophenon, 4,4'-Dihydroxydiphenylsulfon und 4,4'-Dichlordiphenylsulfon

In analoger Weise wie in Beispiel 1 wird ein Polyethersulfon als 4,4'-Dihydroxy-3,3'-diphenylisophthalophenon (0,0501 Mol), 4,4'-Dihydroxydiphenylsulfon (0,0501 Mol) und 4,4'-Dichlordiphenylsulfon (0,1001 Mol) mit Kaliumcarbonat (0,1050 Mol) hergestellt (Reaktionsbedingungen: 1 h/250°C, 1 h/275°C, 4 h/300°C). Das resultierende Polymer mit einer red. Viskosität von 0,57 dl/g besitzt eine Glasübergangstemperatur von 206°C. Es ist zu mehr als 25 % in Methylenchlorid löslich.

Beispiel 4: Polyetherketonsulfon aus 4,4'-Dihydroxy-3,3'-diphenylterephthalophenon und 4,4'-Dichlordiphenylsulfon

In analoger Weise wie in Beispiel 1 wird ein Polyethersulfon aus 4,4'-Dihydroxy-3,3'-diphenylterephthalophenon (0,0502 Mol) und 4,4'-Dichlordiphenylsulfon (0,0501 Mol) mit Kaliumcarbonat (0,0530 Mol) hergestellt (Reaktionsbedingungen: 1 h/250°C, 1 h/277°C, 4 h/300°C). Das resultierende Polymer mit einer red. Viskosität von 0,40 dl/g besitzt eine Glasübergangstemperatur von 191°C. Es ist zu mehr als 25 % in Methylenchlorid löslich.

Beispiel 5: Polyetherketonsulfon-Copolymer aus 4,4'-Dihydroxy-3,3'-diphenylterephthalophenon, 4,4'-Dihydroxydiphenylsulfon und 4,4'-Dichlordiphenylsulfon

In analoger Weise wie in Beispiel 1 wird ein Polyethersulfon aus 4,4'-Dihydroxy-3,3'-diphenylterephthalophenon (0,0251 Mol), 4,4'-Dihydroxydiphenylsulfon (0,0754 Mol) und 4,4'-Dichlordiphenylsulfon (0,1001 Mol) mit Kaliumcarbonat (0,1060 Mol) hergestellt (Reaktionsbedingungen: 1 h/250°C, 4 h/280°C). Das resultierende Polymer mit einer red. Viskosität von 0,38 dl/g besitzt eine Glasübergangstemperatur von 217°C. Es ist zu mehr als 25 % in Methylenchlorid löslich.

Beispiel 6: Polyetherketonsulfon-Copolymeres aus 4,4'-Dihydroxy-3,3'-diphenylterephthalophenon, 4,4'-Dihydroxydiphenylsulfon und 4,4'-Dichlordiphenylsulfon

In analoger Weise wie in Beispiel 1 wird ein Polyethersulfon aus 4,4'-Dihydroxy-3,3'-diphenylterephthalophenon (0,0217 Mol), 4,4'-Dihydroxydiphenylsulfon (0,1946 Mol) und 4,4'-Dichlordiphenylsulfon (0,2163 Mol) mit Kaliumcarbonat (0,2286 Mol) hergestellt (Reaktionsbedingungen: 1 h/253°C, 1 h/272°C und 3,5 h/280°C). Das resultierende Polymer mit einer red. Viskosität von 0,82 dl/g besitzt eine Glasübergangstemperatur von 228°C. Es ist zu mehr als 25 % in Methylenchlorid löslich.

Beispiel 7: Polyester aus 4,4'-Dihydroxy-3,3'-diphenylterephthalophenon und 4,4'-Isophthalsäuredichlorid

Die Herstellung eines Polyesters erfolgt durch umgekehrte Grenzflächen-Polykondensation, ein Verfahren, das beispielsweise in "Methoden der organischen Chemie (Houben/Weyl), Bd. E 20 (Makromolekulare Stoffe), Stuttgart 1987, S. 1420" beschrieben ist.

In einem Rundkolben werden in 250 ml Methylenchlorid 7,15 g (0,0351 Mol) Isophthalsäuredichlorid und 0,23 g (0,001 Mol) Benzyl-triethylammoniumchlorid gelöst. Dazu wird eine Lösung von 16,52 g (0,0351 Mol) 4,4'-Dihydroxy-3,3'-diphenylterephthalophenon in verdünnter Natronlauge (hergestellt aus 3,04 g Natriumhydroxid und 261,3 g dest. Wasser) bei einer Temperatur von -3,5°C unter starkem Rühren im Verlauf von 30 Minuten zugetropft, wobei sich ein weisser Niederschlag bildet. Nach vollständiger Zugabe wird die Mischung bei Raumtemperatur weitere 5 Stunden gerührt und schliesslich über Nacht bei Raumtemperatur stehen gelassen. Zur Isolierung des Polymeren wird das Reaktionsgemisch mit 500 ml Wasser verdünnt und in etwa 15 Liter Wasser gefällt. Das ausgefallene Polymer wird filtriert, mit Wasser und Isopropanol gewaschen und bei 100°C im Vakuumtrockenschrank getrocknet. Es besitzt eine red. Viskosität (1 g Polymer in 100 NMP bei 25°C) von 0,29 dl/g und eine im Differential Scanning Calorimeter bestimmte Glasübergangstemperatur von 188°C.

Beispiele 8-12:

Die in der folgenden Tabelle 1 zusammengestellten Beispiele 8-12 wurden analog Beispiel 1 synthetisiert. Die Eigenschaften der erhaltenen Polyarylenether sind ebenfalls in Tabelle 1 angegeben.

Tabelle 1: Zusammensetzung und Eigenschaften von Polyaryleneetherketonen

| Beispiel | Zusammensetzung | Reaktionsbedingungen | reduzierte Viskosität [dl/g] | Glasübergangstemperatur (DSC) [°C] | Löslichkeit in $CH_2Cl_2$ [%] |
|---|---|---|---|---|---|
| 8 | 4,4'-Dihydroxy-3,3'-diphenyl-isophthalophenon (0.0501 Mol)<br>4,4'-Dihydroxydiphenylsulfon (0.0502 Mol)<br>4,4'-Dihydroxybiphenyl (0.1010 Mol)<br>4,4'-Dichlordiphenylsulfon (0.2001 Mol)<br>Kaliumcarbonat (0.2213 Mol) | 1 h/226°C<br>1 h/248°C<br>3 h/280°C | 0.16 | 179 | >25 |
| 9 | 4,4'-Dihydroxy-3,3'-diphenyl isophthalophenon (0.0200 Mol)<br>4,4'-Dihydroxydiphenylsulfon (0.1610 Mol)<br>4,4'-Dihydroxybenzophenon (0.0200 Mol)<br>4,4'-Dichlordiphenylsulfon (0.2001 Mol)<br>Kaliumcarbonat (0.2205 Mol) | 1 h/227°C<br>1 h/248°C<br>3 h/283°C | 0.50 | 225 | >25 |

| Beispiel | Zusammensetzung | Reaktionsbedingungen | reduzierte Viskosität [dl/g] | Glasübergangstemperatur (DSC) [°C] | Löslichkeit in $CH_2Cl_2$ [%] |
|---|---|---|---|---|---|
| 10 | 4,4'-Dihydroxy-3,3'-diphenyl-isophthalophenon (0.0400 Mol)<br>9,9-Bis(4-hydroxyphenyl)fluoren (0.1606 Mol)<br>4,4'-Dichlordiphenylsulfon (0.2001 Mol)<br>Kaliumcarbonat (0.2230 Mol) | 1 h/227°C<br>1 h/256°C<br>4 h/283°C | 0.15 | 214 | >25 |
| 11 | 4,4'-Dihydroxy-3,3'-diphenyl isophthalophenon (0.0150 Mol)<br>6,6'-Dihydroxy-3,3,3',3'-tetra-methyl-1,1'-spirobiindan (0.0150 Mol)<br>4,4'-Dihydroxydiphenylsulfon (0.1709 Mol)<br>4,4'-Dichlordiphenylsulfon (0.2001 Mol)<br>Kaliumcarbonat (0.2225 Mol) | 1 h/226°C<br>1 h/254°C<br>3 h/284°C | 0.26 | 214 | >25 |

| Beispiel | Zusammensetzung | Reaktionsbedingungen | reduzierte Viskosität [dl/g] | Glasübergangstemperatur (DSC) [°C] | Löslichkeit in $CH_2Cl_2$ [%] |
|---|---|---|---|---|---|
| 12 | 4,4'-Dihydroxy-3,3'-diphenyl-isophthalophenon (0.0502 Mol)<br>2,6-Difluorbenzonitril (0.0505 Mol)<br>Kaliumcarbonat (0.0529 Mol) | 1 h/228°C<br>1 h/253°C<br>3 h/282°C | 0.43 | 186 | >25 |

Anwendunpsbeispiel: 30 Gewichtsteile des gemäss Beispiel 6 hergestellten Polyetherketonsulfoncopolymeren werden als Lösung in Methylenchlorid zu einer Mischung, bestehend aus 50 Gewichtsteilen Tetraglycidyldiaminodiphenylmethan und 50 Gewichtsteilen Triglycidyl-p-aminophenol gegeben und das Lösungsmittel wird im Vakuum entfernt. Nach Zugabe von 50 Gewichtsteilen p-Diaminodiphenylsulfon wird das Gemisch in einer Form 2 Stunden bei 160°C sowie 2 Stunden bei 210°C ausgehärtet. Aus einer so hergestellten Platte werden Prüfkörper geschnitten und die Biegefestigkeit und Randfaserdehnung nach ISO 178 bestimmt.

Biegefestigkeit = 170 $N/mm^2$

Randfaserdehnung = 6,4 %.

**Patentansprüche**

1. Im wesentlichen lineare Polymere mit einer reduzierten Viskosität von 0,1 bis 2,0 dl/g, gemessen an einer 1 gew.-%igen Lösung in N-Methylpyrrolidon (NMP) bei 25°C, enthaltend mindestens einen die Löslichkeit des Polymeren in organischen Lösungsmitteln erhöhenden Anteil an Struktureinheiten der Formel I

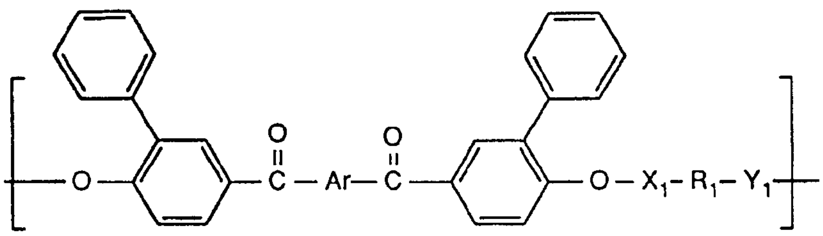 (I),

worin die aromatischen Ringe unsubstituiert oder durch ein oder mehrere $C_1$-$C_4$-Alkyle, $C_1$-$C_4$-Alkoxy oder Halogenatome substituiert sind,
Ar einen zweiwertigen aliphatischen, cycloaliphatischen, araliphatischen oder aromatischen Rest mit bis zu 50 C-Atomen bedeutet,
$X_1$ für eine direkte Bindung, $-SO_2-$, -CO-, -CO-O-, -CO-NH- oder

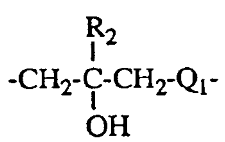

steht, worin $R_2$ ein H-Atom oder Methyl und $Q_1$ -O-, -NH-, -S- oder -OCO- bedeuten,
$Y_1$ für eine direkte Bindung, $-SO_2-$, -CO-, -O-CO-, -NH-CO- oder

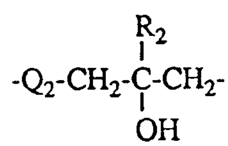

steht, worin $R_2$ ein H-Atom oder Methyl und $Q_2$ -O-, -NH-, -S- oder -OCO- bedeuten,
$R_1$ einen zweiwertigen aliphatischen, cycloaliphatischen, aromatischen oder araliphatischen Rest mit bis zu 30 C-Atomen bedeutet, der unsubstituiert ist oder durch ein oder mehrere $C_1$-$C_4$-Alkyle, $C_1$-$C_4$-Alkylthio, $C_2$-$C_6$-Alkenyle, $C_5$-$C_{12}$-Cycloalkyle, $C_6$-$C_{12}$-Aryle oder eine oder mehrere Cyano- oder Nitrogruppen substituiert ist und in dem ein oder mehrere Kohlenstoffatome durch Sauerstoff-, Schwefel- und/oder Stickstoffatome ersetzt sein können.

2. Polymere gemäss Anspruch 1, worin Ar in Formel I für einen aromatischen Rest steht.

3. Polymere gemäss Anspruch 2, worin Ar einen unsubstituierten oder durch ein oder mehrere $C_1$-$C_4$-Alkyle, $C_1$-$C_4$-Alkoxy oder Halogenatome substituierten Rest der Formeln

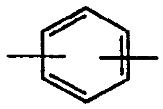, 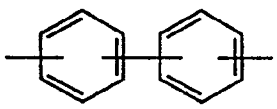, 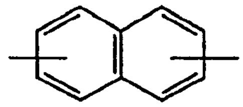, 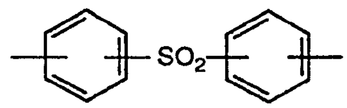,

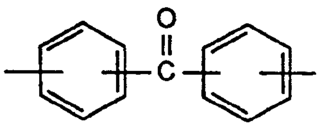 oder 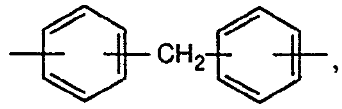,

bedeutet.

4. Polymere gemäss Anspruch 1, enthaltend, bezogen auf die Gesamtmenge der im Polymer vorhandenen Struktureinheiten, 5 bis 100 Mol-% einer wiederkehrenden Struktweinheit der Formel I und 95 bis 0 Mol-% einer wiederkehrenden Struktureinheit der Formel II

$$\{O\text{-}R_3\text{-}O\text{-}X_1\text{-}R_1\text{-}Y_1\} \quad (II)$$

worin $X_1$, $R_1$ und $Y_1$ die gleiche Bedeutung wie in Formel I haben und $R_3$ einen zweiwertigen aliphatischen, cycloaliphatischen, araliphatischen oder aromatischen Rest mit bis zu 50 C-Atomen bedeutet, der vom Rest

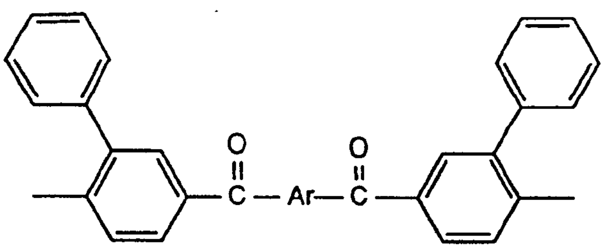

verschieden ist, worin Ar die gleiche Bedeutung wie in Formel I hat.

5. Polymere gemäss Anspruch 4, worin $R_3$ in Formel II einen unsubstituierten oder durch ein oder mehrere $C_1$-$C_4$-Alkyle, $C_1$-$C_4$-Alkoxy oder Halogenatome substituierten Rest der Formeln IIIa bis IIIg bedeutet

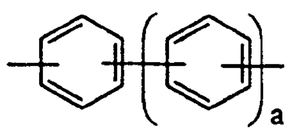 (IIIa),

wobei a Null oder die Zahl 1 ist,

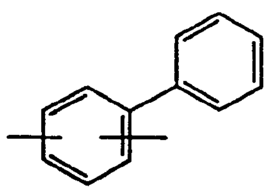 (IIIb), 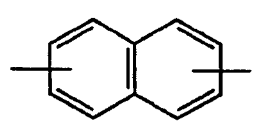 (IIIc),

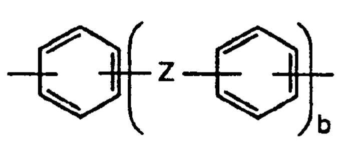 (IIId),

wobei b die Zahl 1 oder 2 ist,

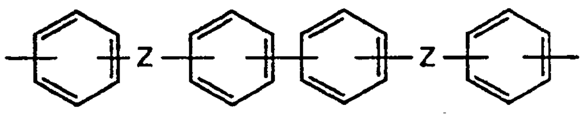 (IIIe), 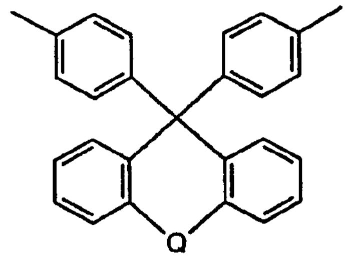 (IIIf)

oder

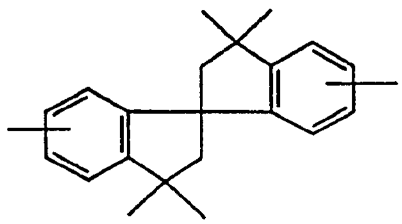 (IIIg)

darstellt, worin Z für -CO-, $-SO_2-$, -SO-, -S-, -O-,

$-C(CH_3)_2$ , $-C(CF_3)_2$ , ·

$-CH_2-$,

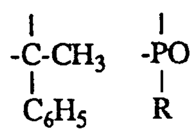

steht, wobei R ein Methyl oder Phenyl bedeutet und Q für $-CH_2-$, -O-,

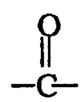 ,

oder die direkte Bindung steht.

6. Polymere gemäss Anspruch 1 enthaltend, bezogen auf die Gesamtmenge der im Polyarylenetherharz vorhandenen Struktureinheiten, 5-100 Mol-% einer wiederkehrenden Struktureinheit der Formel IV

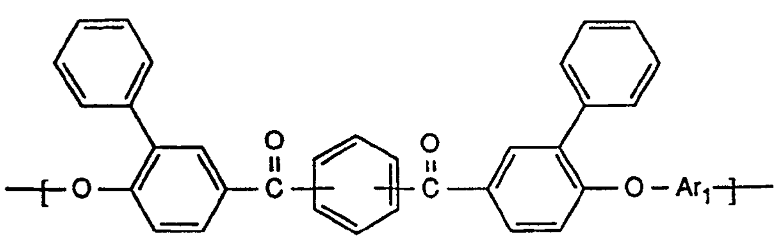 (IV)

und 95-0 Mol-% einer wiederkehrenden Struktureinheit der Formel V

$[O-Ar_2-O-Ar_1]$ (V)

worin die beiden Carbonylgruppen zueinander in para- oder meta-Stellung stehen, $Ar_1$ einen unsubstituierten oder durch ein oder mehrere $C_1$-$C_4$-Alkyle, $C_1$-$C_4$-Alkoxy oder Halogenatome substituierten Rest der Formel IVa bis IVe

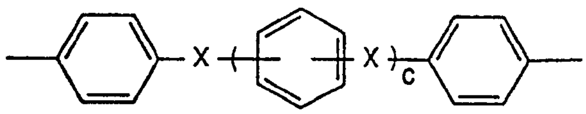 (IVa),

wobei c für Null oder die Zahl 1 oder 2 steht, oder

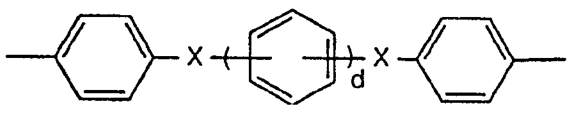 (IVb),

wobei d die Zahl 2 oder 3 bedeutet,

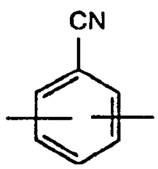 (IVc), 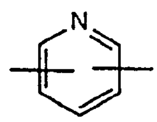 (IVd) oder 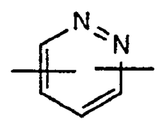 (IVe)

darstellt, worin X für -CO-, $-SO_2-$ oder -SO- steht und $Ar_2$ einen unsubstituierten oder durch ein oder mehrere $C_1$-$C_4$-Alkyle, $C_1$-$C_4$-Alkoxy oder Halogene substituierten Rest der Formeln Va-Vg

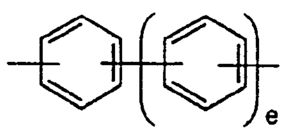 (Va),

wobei e Null oder die Zahl 1 bedeutet,

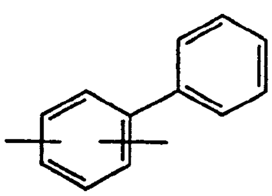 (Vb), 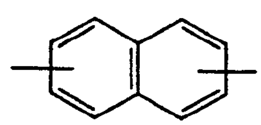 (Vc),

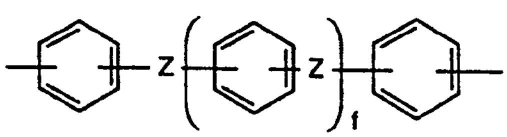 (Vd),

wobei f Null oder die Zahl 1 ist,

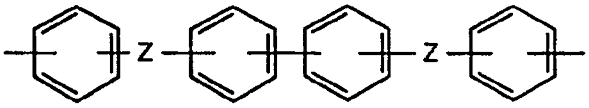 (Ve), 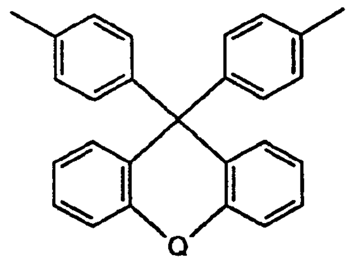 (Vf)

oder

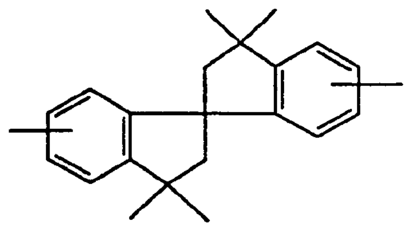 (Vg)

darstellt, worin Z für -CO-, $SO_2$-, -SO-, -S-, -O-,

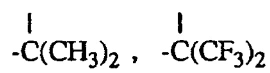 ,

-$CH_2$-,

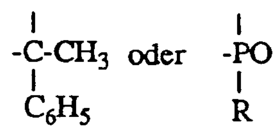

steht, worin R ein Methyl oder Phenyl bedeutet und Q für -$CH_2$-, -O-,

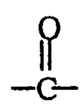

oder die direkted Bindung steht.

7. Polymere gemäss Anspruch 6, enthaltend 10-100 Mol-% einer wiederkehrenden Struktureinheit der Formel IV und 90-0 Mol-% einer wierderkehrenden Struktureinheit der Formel V, worin die beiden Carbonylgruppen zueinander in meta- oder para-Stellung stehen, $Ar_1$ einen Rest der Formeln

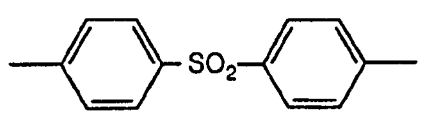 ,

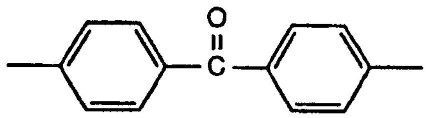 oder 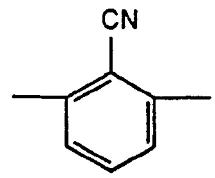

bedeutet und $Ar_2$ für einen Rest der Formeln

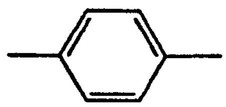 , 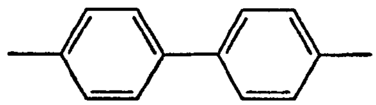 , 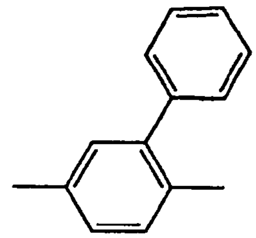 ,

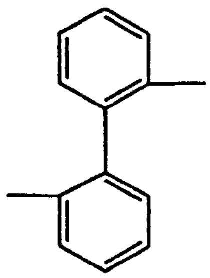 , 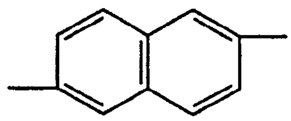 , 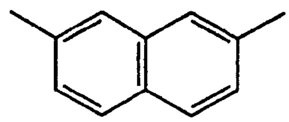 ,

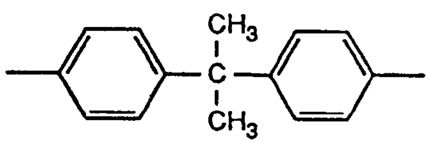 , 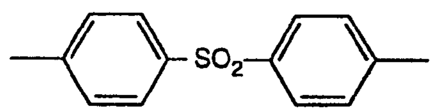 ,

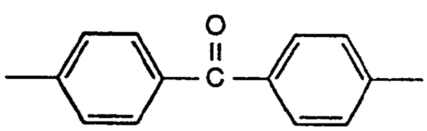 , 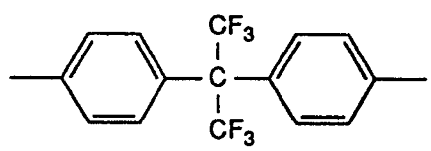 ,

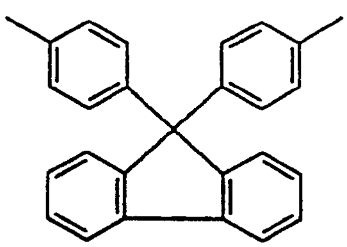 oder 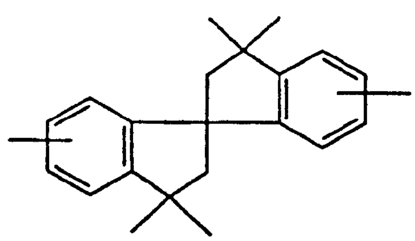 steht.

8. Lösung enthaltend 1 bis 75 Gew.-%, bezogen auf die Lösung eines Polymeren gemäss Anspruch 1, gelöst in einem organischen Lösungsmittel.

9. Verfahren zur Herstellung von Polymeren gemäss Anspruch 1, dadurch gekennzeichnet, dass man ein unsubstituiertes oder durch ein oder mehere $C_1$-$C_4$-Alkyle, $C_1$-$C_4$-Alkoxy oder Halogenatome substituiertes Diphenol der Formel VI

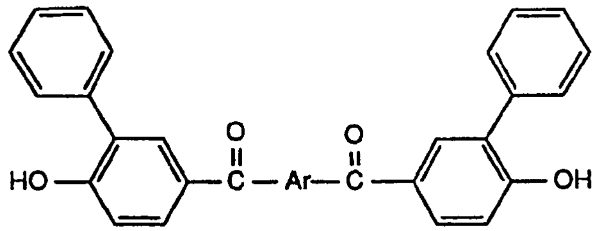

(VI),

worin Ar die gleiche Bedeutung wie in Formel I hat, oder ein Gemisch aus einem Diphenol der Formel VI und einer darin bis zu 95 Mol-% enthaltenen, vom Diphenol der Formel VI verschiedenen Dihydroxyverbindung in äquimolaren Mengen mit einer Verbindung der Formel VII

$$Z_1\text{-}R_1\text{-}Z_2 \qquad (VII),$$

umsetzt, worin $R_1$ die gleiche Bedeutung wie in Formel I hat und $Z_1$ und $Z_2$ unabhängig voneinander je ein Halogenatom, vorzugsweise Fluor- oder Chloratom, oder einen Rest der Formeln $-SO_3H$, $-SO_3R_4$, $-SO_2Cl$, -COOH, -COCl, $-COOR_4$-, $-OR_4$, -O-COCl, -N=C=O,

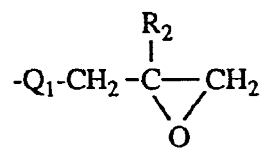 oder 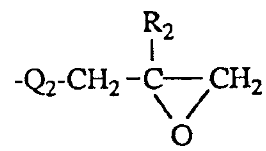

bedeuten, worin $R_2$ für ein H-Atom oder Methyl und $R_4$ für ein $C_1$-$C_4$-Alkyl oder Phenyl stehen und $Q_1$ und $Q_2$ -O-, -NH-, -S- oder -OCO- bedeuten.

10. Unsubstituiertes oder durch ein oder mehrere $C_1$-$C_4$-Alkyle, $C_1$-$C_4$-Alkoxy oder Halogenatome substituiertes Diphenol der Formel VI

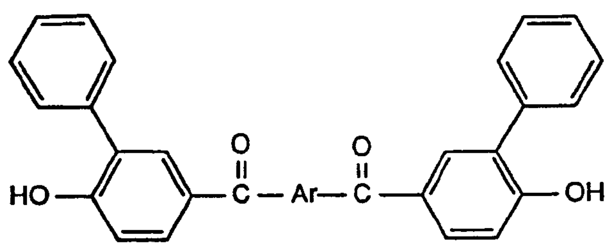

(VI),

worin Ar die gleiche Bedeutung wie in Formel I gemäss Anspruch 1 hat.

11. Unsubstituierte oder durch ein oder mehrere $C_1$-$C_4$-Alkyle, $C_1$-$C_4$-Alkoxy oder Halogenatome substituierte Verbindung der Formel IX

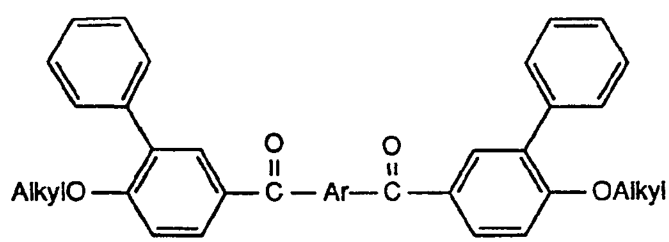

(IX),

worin Ar die gleiche Bedeutung wie in Formel I gemäss Anspruch 1 hat und Alkyl für ein $C_1$-$C_4$-Alkyl steht.

12. Unsubstituierte oder durch ein oder mehrere $C_1$-$C_4$-Alkyle, $C_1$-$C_4$-Alkoxy oder Halogenatome substituierte Dihalogenverbindung der Formel XI

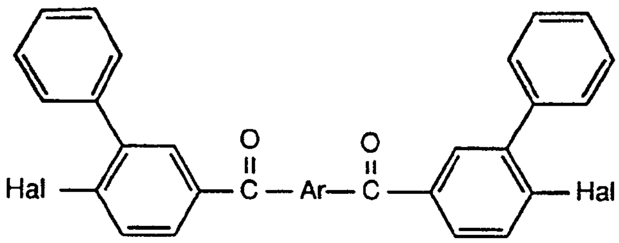

(XI),

worin Ar die gleiche Bedeutung wie in Formel I gemäss Anspruch 1 hat und Hal für ein Halogenatom steht.

13. Verbindungen der Formel XII

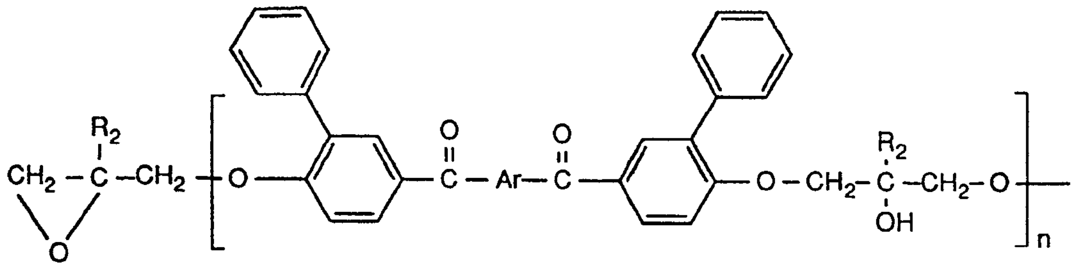

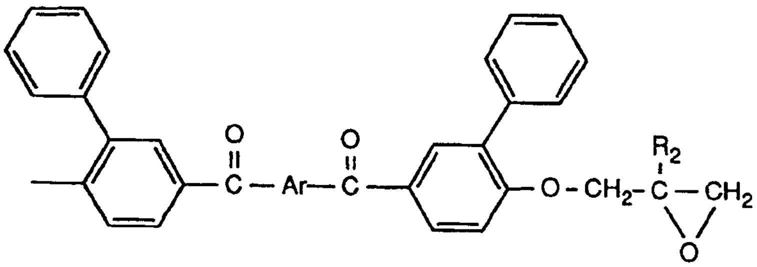

(XII),

worin Ar die gleiche Bedeutung wie in Formel I gemäss Anspruch 1 hat, $R_2$ für ein H-Atom oder Methyl steht und n Null oder eine Zahl von 1 bis 20 ist.

14. Formstoffe, Beschichtungen oder Folien enthaltend ein Polymer gemäss Anspruch 1.

15. Verwendung der Polymere gemäss Anspruch 1 zum Modifizieren von thermoplastischen und duromeren Matrixharzen.

**Patentansprüche für folgenden Vertragsstaat : ES**

1. Verfahren zur Herstellung von im wesentlichen linearen Polymeren mit einer reduzierten Viskosität von 0,1 bis 2,0 dl/g, gemessen an einer 1 gew.-%igen Lösung in N-Methylpyrrolidon (NMP) bei 25°C, enthaltend mindestens einen die Löslichkeit des Polymeren in organischen Lösungsmitteln erhöhenden Anteil an Struktureinheiten der Formel I

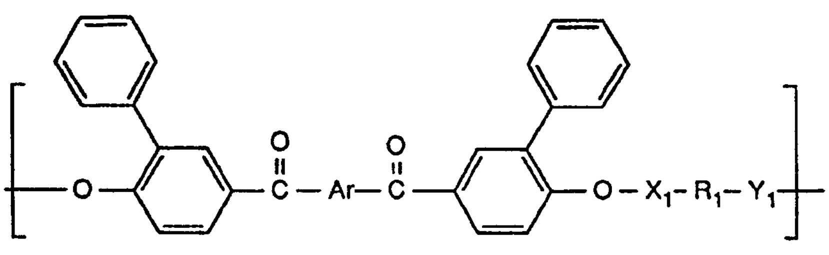

(I),

worin die aromatischen Ringe unsubstituiert oder durch ein oder mehrere $C_1$-$C_4$-Alkyle, $C_1$-$C_4$-Alkoxy oder Halogenatome substituiert sind,
Ar einen zweiwertigen aliphatischen, cycloaliphatischen, araliphatischen oder aromatischen Rest mit bis zu 50 C-Atomen bedeutet,
$X_1$ für eine direkte Bindung, -$SO_2$-, -CO-, -CO-O-, -CO-NH- oder

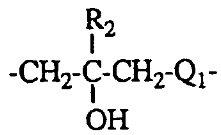

steht, worin $R_2$ ein H-Atom oder Methyl und $Q_1$ -O-, -NH-, -S- oder -OCO- bedeuten,
$Y_1$ für eine direkte Bindung, -$SO_2$-, -CO-, -O-CO-, -NH-CO- oder

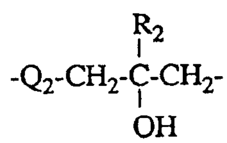

steht, worin $R_2$ ein H-Atom oder Methyl und $Q_2$ -O-, -NH-, -S- oder -OCO- bedeuten,
$R_1$ einen zweiwertigen aliphatischen, cycloaliphatischen, aromatischen oder araliphatischen Rest mit bis zu 30 C-Atomen bedeutet, der unsubstituiert ist oder durch ein oder mehrere $C_1$-$C_4$-Alkyle, $C_1$-$C_4$-Alkylthio, $C_2$-$C_6$-Alkenyle, $C_5$-$C_{12}$-Cycloalkyle, $C_6$-$C_{12}$-Aryle oder eine oder mehrere Cyano- oder Nitrogruppen substituiert ist und in dem ein oder mehrere Kohlenstoffatome durch Sauerstoff-, Schwefel- und/oder Stickstoffatome ersetzt sein können, dadurch gekennzeichnet, dass man ein unsubstituiertes oder durch ein oder mehrere $C_1$-$C_4$-Alkyle, $C_1$-$C_4$-Alkoxy oder Halogenatome substituiertes Diphenol der Formel VI

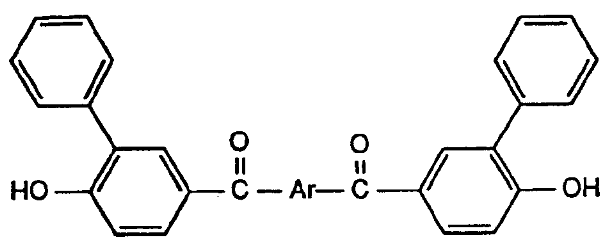

(VI),

worin Ar die gleiche Bedeutung wie in Formel I hat, oder ein Gemisch aus einem Diphenol der Formel VI und einer darin bis zu 95 Mol-% enthaltenen, vom Diphenol der Formel VI verschiedenen Dihydroxyverbindung in äquimolaren Mengen mit einer Verbindung der Formel VII

$Z_1$-$R_1$-$Z_2$ (VII),

worin $R_1$ die gleiche Bedeutung wie in Formel I hat und $Z_1$ und $Z_2$ unabhängig voneinander je ein Halogenatom, vorzugsweise Fluor- oder Chloratom, oder einen Rest der Formeln -$SO_3H$, -$SO_3R_4$, -$SO_2Cl$, -CO-OH, -COCl, -$COOR_4$ , -$OR_4$, -O-COCl, -N=C=O,

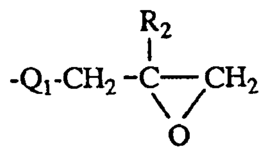 oder 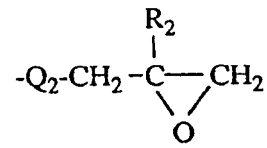

bedeuten, worin $R_2$ für ein H-Atom oder Methyl und $R_4$ für ein $C_1$-$C_4$-Alkyl oder Phenyl stehen und $Q_1$ und $Q_2$ -O-, -NH-, -S- oder -OCO- bedeuten, umsetzt, bis der erhaltene Polyarylenether eine reduzierte Viskosität von 0,1 bis 2,0 dl/g aufweist.

2. Verfahren zur Herstellung von im wesentlichen linearen Polymeren, enthaltend mindestens einen die Löslichkeit des Polymeren in organischen Lösungsmitteln erhöhen den Anteil an Struktureinheiten der Formel

I gemäss Anspruch 1, dadurch gekennzeichnet, dass man (a) ein Diphenol der Formel VI, ein Gemisch aus einem Diphenol der Formel VI oder dessen Alkali- oder Erdalkaliphenolat und einer darin bis zu 95 Mol-% enthaltenen Dihydroxyverbindung der Formel X

$$HO\text{-}Ar_2\text{-}OH \quad (X)$$

oder deren Alkali- oder Erdalkalisalze, worin $Ar_2$ einen unsubstituierten oder durch ein oder mehrere $C_1$-$C_4$-Alkyle, $C_1$-$C_4$-Alkoxy oder Halogene substituierten Rest der Formeln Va-Vg

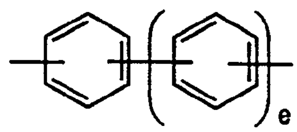 (Va),

wobei e Null oder die Zahl 1 bedeutet,

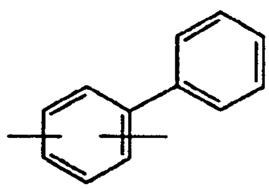 (Vb), 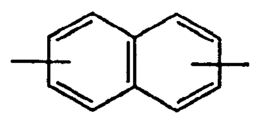 (Vc),

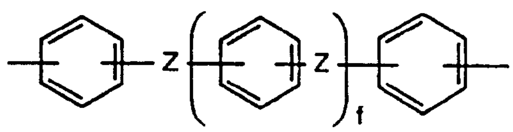 (Vd),

wobei f Null oder die Zahl 1 ist,

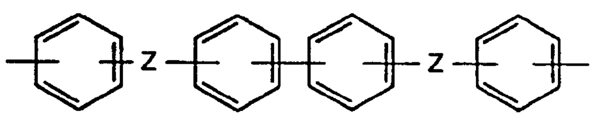 (Ve), 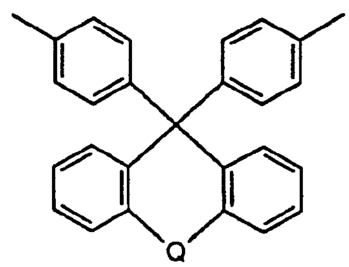 (Vf)

oder

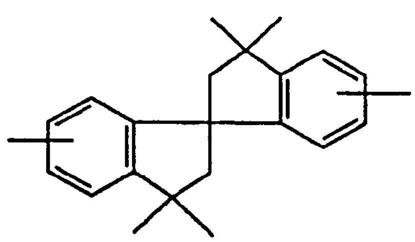 (Vg)

darstellt, worin Z für -CO-, $-SO_2-$, -SO-, -S-, -O-,

$$-\overset{|}{C}(CH_3)_2\ ,\quad -\overset{|}{C}(CF_3)_2\ ,$$

$-CH_2-$,

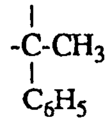 oder 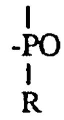

steht, worin R ein Methyl oder Phenyl bedeutet und Q für $-CH_2-$, -O-,

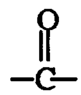

oder die direkte Bindung steht,
(b) eine unsubstituierte oder durch ein oder mehrere $C_1$-$C_4$-Alkyle, $C_1$-$C_4$-Alkoxy oder Halogenatome substituierte Dihalogenverbindung der Formel XI

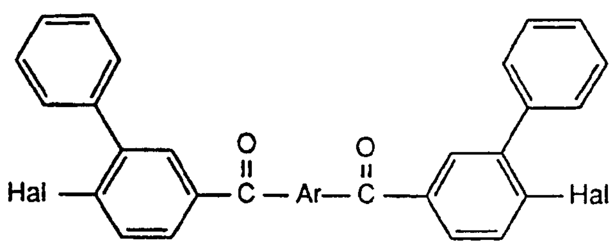

(XI),

worin Ar die gleiche Bedeutung wie in Formel I hat und Hal für ein Halogenatom steht, oder eines Gemisches aus einer Dihalogenverbindung der Formel XI und einer darin bis zu 95 Mol-% enthaltenen, von der Dihalogenverbindung der Formel XI verschiedenen Dihalogenverbindung der Formel VII in äquimolaren Mengen mit einer Dihydroxyverbindung der Formel X oder deren Alkali- oder Erdalkalisalze in Gegenwart von Alkali in einem aprotischen Lösungsmittel polykondensiert, bis der erhaltene Polyarylenether eine reduzierte Viskosität von 0,1 bis 2,0 dl/g aufweist.